# EUROPEAN PATENT SPECIFICATION

(11) **EP 1 790 318 B1**
(45) Date of publication and mention of the grant of the patent: **22.04.2009**
(21) Application number: 06255867.1
(22) Date of filing: 16.11.2006
(51) Int. Cl.: A61F 2/24

(54) **Magnetic engagement of catheter to implantable device**
Magnetische Befestigung eines Katheters an ein Implantat
Embrayage magnétique entre cathéter et implant

(30) Priority: 16.11.2005 US 737104 P
(43) Date of publication of application: 30.05.2007
(73) Proprietor: Micardia Corporation, Irvine CA 92618 (US)
(72) Inventor: Moaddeb, Shahram, Irwine, CA 92620 (US)
(74) Representative: Hill, Justin John

(56) References cited:
- WO-A-20/04019826
- WO-A2-20/05062931
- US-A1- 2003 191 528
- US-A1- 2004 243 227

## Description

### Background of the Invention

### Field of the Invention

The present invention relates to devices for reinforcing dysfunctional heart valves and other body structures. More specifically, the present invention relates to annuloplasty rings that can be adjusted within the body of a patient.

### Description of the Related Art

The circulatory system of mammals includes the heart and the interconnecting vessels throughout the body that include both veins and arteries. The human heart includes four chambers, which are the left and right atrium and the left and right ventricles. The mitral valve, which allows blood flow in one direction, is positioned between the left ventricle and left atrium. The tricuspid valve is positioned between the right ventricle and the right atrium. The aortic valve is positioned between the left ventricle and the aorta, and the pulmonary valve is positioned between the right ventricle and pulmonary artery. The heart valves function in concert to move blood throughout the circulatory system. The right ventricle pumps oxygen-poor blood from the body to the lungs and then into the left atrium. From the left atrium, the blood is pumped into the left ventricle and then out the aortic valve into the aorta. The blood is then recirculated throughout the tissues and organs of the body and returns once again to the right atrium.

If the valves of the heart do not function properly, due either to disease or congenital defects, the circulation of the blood may be compromised. Diseased heart valves may be stenotic, wherein the valve does not open sufficiently to allow adequate forward flow of blood through the valve, and/or incompetent, wherein the valve does not close completely. Incompetent heart valves cause regurgitation or excessive backward flow of blood through the valve when the valve is closed. For example, certain diseases of the heart valves can result in dilation of the heart and one or more heart valves. When a heart valve annulus dilates, the valve leaflet geometry deforms and causes ineffective closure of the valve leaflets. The ineffective closure of the valve can cause regurgitation of the blood, accumulation of blood in the heart, and other problems.

Diseased or damaged heart valves can be treated by valve replacement surgery, in which damaged leaflets are excised and the annulus is sculpted to receive a replacement valve. Another repair technique that has been shown to be effective in treating incompetence is annuloplasty, in which the effective size of the valve annulus is contracted by attaching a prosthetic annuloplasty repair segment or ring to an interior wall of the heart around the valve annulus. The annuloplasty ring reinforces the functional changes that occur during the cardiac cycle to improve coaptation and valve integrity. Thus, annuloplasty rings help reduce reverse flow or regurgitation while permitting good hemodynamics during forward flow.

Generally, annuloplasty rings comprise an inner substrate of a metal such as stainless steel or titanium, or a flexible material such as silicon rubber or Dacron@. The inner substrate is generally covered with a biocompatible fabric or cloth to allow the ring to be sutured to the heart tissue. Annuloplasty rings may be stiff or flexible, may be open or closed, and may have a variety of shapes including circular, D-shaped, or C-shaped. The configuration of the ring is generally based on the shape of the heart valve being repaired or on the particular application. For example, the tricuspid valve is generally circular and the mitral valve is generally D-shaped. Further, C-shaped rings may be used for tricuspid valve repairs, for example, because it allows a surgeon to position the break in the ring adjacent the atrioventricular node, thus avoiding the need for suturing at that location.

Annuloplasty rings support the heart valve annulus and restore the valve geometry and function. Although the implantation of an annuloplasty ring can be effective, the heart of a patient may change geometry over time after implantation. For example, the heart of a child will grow as the child ages. As another example, after implantation of an annuloplasty ring, dilation of the heart caused by accumulation of blood may cease and the heart may begin returning to its normal size. Whether the size of the heart grows or reduces after implantation of an annuloplasty ring, the ring may no longer be the appropriate size for the changed size of the valve annulus.

WO 2004/019826 discloses implants which can be adjusted to alter and maintain the size of an orifice to which they are attached.

US 2003/191528 discloses an expandable annular ring for implantation in a valvular annulus.

### Summary of the Invention

Thus, it would be advantageous to develop systems for reinforcing a heart valve annulus or other body structure using an annuloplasty device that can be adjusted within the body of a patient in a minimally invasive or non-invasive manner.

In one embodiment, an annuloplasty activation system for percutaneously activating an adjustable annuloplasty device is disclosed. The system comprises an elongate member having a proximal end and distal end, the distal end configured to be inserted percutaneously into a vein of a patient, further configured to be inserted across a fossa ovalis in an interatrial septum of a patient, and further configured to be inserted into the left atrium of the patient while the proximal end is located outside the patient's body. The system further comprises a distal element at the distal end of the elongate member, the distal element configured to emanate a magnetic field within the left atrium, configured to magnetically attach to the annuloplasty device and further configured to deliver energy through the distal end to the annuloplasty device; wherein the distal element comprises at least one of a permanent magnet and an electromagnet, the electromagnet being activatable from outside the patient's body; and wherein the adjustable annuloplasty device is adjusted by applying an activation energy through the distal element to activate a shape memory material in the adjustable annuloplasty device and cause it to change to a memorized shape

In certain embodiments, the annuloplasty device, comprises a body member having a proximal end, a distal end and a length extending therebetween, the body member configured to be implanted within a patient's heart at or near a base of a heart valve; wherein the body member comprises a first portion, comprising the shape memory material and being transformable from a first configuration to a second configuration in response to the activation energy; and a second portion coupled to the first portion, the second portion comprising a magnetic material that is responsive to the magnetic field. When in position at or near the base of the heart valve and when the body member transforms from the first configuration to the second configuration, the body member reshapes a tissue of the heart so as to exert a force on the heart valve base.

In certain embodiments, the distal element is further configured to deliver electric field energy, thermal energy, magnetic field energy, radio frequency energy, x-ray energy, microwave energy, ultrasonic energy, light energy, or combinations of the foregoing.

In certain embodiments, the distal end comprises a flexible portion.

In certain embodiments, the distal element comprises a plurality of magnetic bands.

In certain embodiments, the elongate member comprises a port at the proximal end in fluid communication with the distal end.

In certain embodiments, the elongate member is configured to deliver energy to the annuloplasty device.

In certain embodiments, the magnetic material produces a force between approximately 0.89 N (0.2 pounds) of force and approximately 8.9 N (2.0 pounds) of force, at a distance of between 1 millimeter to 10 millimeters.

In certain embodiments, the magnetic material produces a magnetic field between approximately 100 Gauss and approximately 10,000 Gauss. In certain embodiments, the magnetic material produces a force in a range between 0.45 N (0.1 pounds) of force and 13.35 N (3.0 pounds) of force at a distance between 0.5 millimeters and twenty millimeters and has a range between approximately 100 Gauss and approximately 700 Gauss.

In certain embodiments, the magnetic material comprises cylindrical shaped magnets. In certain embodiments, the magnets have a diameter between approximately 0.2 mm and approximately 0.4 mm, and a height between approximately 0.2 mm and approximately 0.4 mm.

In certain embodiments, the magnetic material comprises a rod shape, a spherical shape, a disk shape, a cubic shape, or a band shape.

In certain embodiments, the annuloplasty device is ring shaped. In certain embodiments, the annuloplasty device is C-shaped. In certain embodiments, the annuloplasty device is D-shaped.

For purposes of summarizing the invention, certain aspects, advantages, and novel features of the invention have been described herein. It is to be understood that not necessarily all such advantages may be achieved in accordance with any particular embodiment of the invention. Thus, the invention may be embodied or carried out in a manner that achieves or optimizes one advantage or group of advantages as taught herein without necessarily achieving other advantages as may be taught or suggested herein.

### Brief Description of the Drawings

Systems and methods which embody the various features of the invention will now be described with reference to the following drawings:

FIG. 1A is a top view in partial section of an adjustable annuloplasty ring;

FIG. 1B is a side view of the annuloplasty ring of FIG. 1A;

FIG. 1C is a transverse cross-sectional view of the annuloplasty ring of FIG. 1A;

FIG. 2 is a graphical representation of the diameter of an annuloplasty ring in relation to the temperature of the annuloplasty ring;

FIG. 3A is a top view in partial section of an adjustable annuloplasty ring having a D-shaped configuration;

FIG. 3B is a side view of the annuloplasty ring of FIG. 3A;

FIG. 3C is a transverse cross-sectional view of the annuloplasty ring of FIG. 3A;

FIG. 4A is a top view of an annuloplasty ring having a substantially circular configuration;

FIG. 4B is a side view of the annuloplasty ring of FIG. 4A;

FIG. 4C is a transverse cross-sectional view of the annuloplasty ring of FIG. 4A;

FIG. 5 is a top view of an annuloplasty ring having a substantially D-shaped configuration;

FIG. 6A is a schematic diagram of a top view of a shape memory wire having a substantially D-shaped configuration;

FIGS. 6B-6E are schematic diagrams of side views of the shape memory wire of FIG. 6A;

FIG. 7A is a perspective view in partial section of an annuloplasty ring comprising the shape memory wire of FIG. 6A;

FIG. 7B is a perspective view in partial section of a portion of the annuloplasty ring of FIG. 7A;

FIG. 8 is a schematic diagram of a shape memory wire having a substantially C-shaped configuration;

FIG. 9A is a perspective view in partial section of an annuloplasty ring comprising the shape memory wire of FIG. 8;

FIG. 9B is a perspective view in partial section of a portion of the annuloplasty ring of FIG. 9A;

FIG. 10A is a perspective view in partial section an annuloplasty ring comprising a first shape memory wire and a second shape memory wire;

FIG. 10B is a top cross-sectional view of the annuloplasty ring of FIG. 10A;

FIG. 11A is a perspective view in partial section of an annuloplasty ring comprising a first shape memory wire and a second shape memory wire;

FIG. 11B is a top cross-sectional view of the annuloplasty ring of FIG. 11A;

FIG. 12 is a perspective view of a shape memory wire wrapped in a coil;

FIGS. 13A and 13B are schematic diagrams illustrating an annuloplasty ring;

FIG. 14 is a schematic diagram illustrating an annuloplasty ring;

FIG. 15 is a schematic diagram illustrating an annuloplasty ring;

FIGS. 16A and 16B are schematic diagrams illustrating an annuloplasty ring having a plurality of temperature response zones or sections;

FIGS. 17A and 17B are schematic diagrams illustrating an annuloplasty ring having a plurality of temperature response zones or sections;

FIG. 18 is a sectional view of a mitral valve with respect to an exemplary annuloplasty ring;

FIG. 19 is a schematic diagram of a substantially C-shaped wire comprising a shape memory material configured to contract in a first direction and expand in a second direction;

FIGS. 20A and 20B are schematic diagrams of a body member usable by an annuloplasty ring;

FIGS. 21A and 21B are schematic diagrams of a body member usable by an annuloplasty ring;

FIGS. 22A and 22B are schematic diagrams of a body member usable by an annuloplasty ring;

FIG. 23 is a transverse cross-sectional view of the body member of FIGS. 21A and 21B;

FIG. 24 is a perspective view of a body member usable by an annuloplasty ring; comprising a first shape memory band and a second shape memory band;

FIG. 25A is a schematic diagram illustrating the body member of FIG. 24 in a first configuration or shape;

FIG. 25B is a schematic diagram illustrating the body member of FIG. 24 in a second configuration or shape;

FIG. 25C is a schematic diagram illustrating the body member of FIG. 24 in a third configuration or shape;

FIG. 26 is a perspective view illustrating an annuloplasty ring comprising one or more thermal conductors;

FIGS. 27A-27C are transverse cross-sectional views of the annuloplasty ring of FIG. 26 schematically illustrating a device for conducting thermal energy to an internal shape memory wire;

FIG. 28 is a schematic diagram of an annuloplasty ring comprising one or more thermal conductors;

FIG. 29A is a schematic diagram of an annuloplasty ring comprising one or more magnetic devices according to certain embodiments of the invention;

FIG. 29B is a schematic diagram of an annuloplasty ring comprising one or more magnetic bands according to certain embodiments of the invention;

FIG. 30 is a schematic diagram of the body member of FIGS. 20A and 20B further comprising one or more magnetic devices according to certain embodiments of the invention;

FIG. 31 is a partial schematic diagram of a portion of the body member of FIG. 30 further comprising one or more thermal conductors according to certain embodiments of the invention;

FIG. 32 is a schematic diagram of a magnetic tipped catheter according to certain embodiments of the invention;

FIG. 33 is a schematic diagram illustrating one embodiment for aligning an internal shape memory element according to certain embodiments of the invention; and

FIG. 34 is a schematic diagram illustrating one embodiment for conducting thermal energy to an internal shape memory element according to certain embodiments of the invention.

### Detailed Description of the Preferred Embodiment

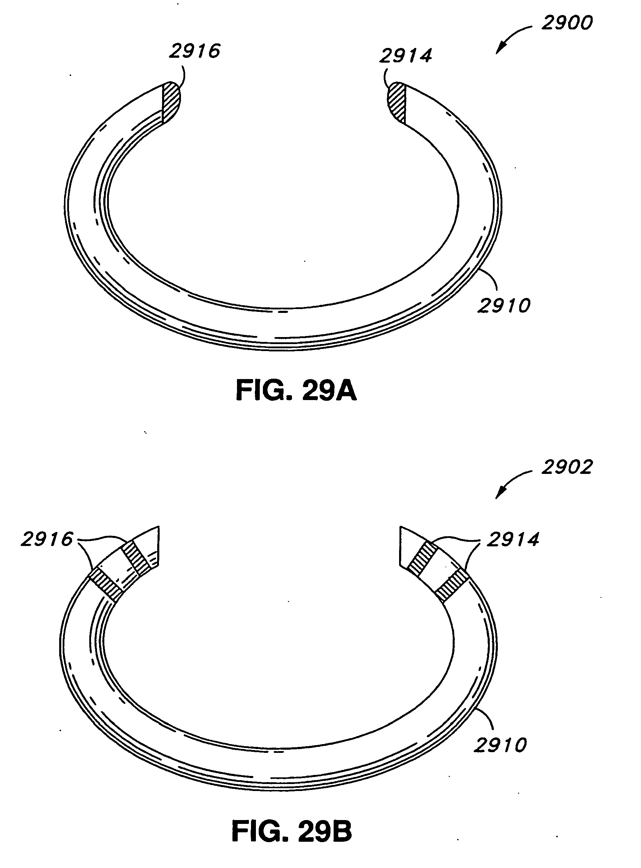

The present invention involves systems for reinforcing dysfunctional heart valves and other body structures with adjustable rings. An adjustable annuloplasty ring is implanted into the body of a patient such as a human or other animal. The invention is solely set out in Figures 29-34 and the respective description thereof. The adjustable annuloplasty ring is implanted through an incision or body opening either thoracically (e.g., open-heart surgery) or percutaneously (e.g., via a femoral artery or vein, or other arteries or veins) as is known to someone skilled in the art. The adjustable annuloplasty ring is attached to the annulus of a heart valve to improve leaflet coaptation and to reduce regurgitation. The annuloplasty ring may be selected from one or more shapes comprising a round or circular shape, an oval shape, a C-shape, a D-shape, a U-shape, an open circle shape, an open oval shape, and other curvilinear shapes.

The size of the annuloplasty ring can be adjusted postoperatively to compensate for changes in the size of the heart. As used herein, "postoperatively" refers to a time after implanting the adjustable annuloplasty ring and closing the body opening through which the adjustable annuloplasty ring was introduced into the patient's body. For example, the annuloplasty ring may be implanted in a child whose heart grows as the child gets older. Thus, the size of the annuloplasty ring may need to be increased. As another example, the size of an enlarged heart may start to return to its normal size after an annuloplasty ring is implanted. Thus, the size of the annuloplasty ring may need to be decreased postoperatively to continue to reinforce the heart valve annulus.

In certain embodiments, the annuloplasty ring comprises a shape memory material that is responsive to changes in temperature and/or exposure to a magnetic field. Shape memory is the ability of a material to regain its shape after deformation. Shape memory materials include polymers, metals, metal alloys and ferromagnetic alloys. The annuloplasty ring is adjusted in vivo by applying an energy source to activate the shape memory material and cause it to change to a memorized shape. The energy source may include, for example, thermal energy, radio frequency (RF) energy, x-ray energy, microwave energy, ultrasonic energy such as focused ultrasound, high intensity focused ultrasound (HIFU) energy, light energy, electric field energy, magnetic field energy, combinations of the foregoing, or the like. For example, one embodiment of electromagnetic radiation that is useful is infrared energy having a wavelength in a range between approximately 750 nanometers and approximately 1600 nanometers. This type of infrared radiation may be produced efficiently by a solid state diode laser. In certain embodiments, the annuloplasty ring implant is selectively heated using short pulses of energy having an on and off period between each cycle. The energy pulses provide segmental heating which allows segmental adjustment of portions of the annuloplasty ring without adjusting the entire implant.

In certain embodiments, the annuloplasty ring includes an energy absorbing material to increase heating efficiency and localize heating in the area of the shape memory material. Thus, damage to the surrounding tissue is reduced or minimized. Energy absorbing materials for light or laser activation energy may include nanoshells, nanospheres and the like, particularly where infrared laser energy is used to energize the material. Such nanoparticles may be made from a dielectric, such as silica, coated with an ultra thin layer of a conductor, such as gold, and be selectively tuned to absorb a particular frequency of electromagnetic radiation. In certain such embodiments, the nanoparticles range in size between about 5 nanometers and about 20 nanometers and can be suspended in a suitable material or solution, such as saline solution. In certain embodiments, the nanoparticles range in size between about 2 nanometers and about 30 nanometers. In certain embodiments, the nanoparticles range in size between about 5 nanometers and about 20 nanometers. In certain embodiments, the nanoparticles range in size between about 8 nanometers and about 15 nanometers. Coatings comprising nanotubes or nanoparticles can also be used to absorb energy from, for example, HIFU, MRI, inductive heating, or the like.

In other embodiments, thin film deposition or other coating techniques such as sputtering, reactive sputtering, metal ion implantation, physical vapor deposition, and chemical deposition can be used to cover portions or all of the annuloplasty ring. Such coatings can be either solid or microporous. When HIFU energy is used, for example, a microporous structure traps and directs the HIFU energy toward the shape memory material. The coating improves thermal conduction and heat removal. In certain embodiments, the coating also enhances radio-opacity of the annuloplasty ring implant. Coating materials can be selected from various groups of biocompatible organic or non-organic, metallic or non-metallic materials such as Titanium Nitride (TiN), Iridium Oxide (Irox), Carbon, Platinum black, Titanium Carbide (TiC) and other materials used for pacemaker electrodes or implantable pacemaker leads. Other materials discussed herein or known in the art can also be used to absorb energy.

In addition, or in other embodiments, fine conductive wires such as platinum coated copper, titanium, tantalum, stainless steel, gold, or the like, are wrapped around the shape memory material to allow focused and rapid heating of the shape memory material while reducing undesired heating of surrounding tissues.

In certain embodiments, the energy source is applied surgically either during implantation or at a later time. For example, the shape memory material can be heated during implantation of the annuloplasty ring by touching the annuloplasty ring with warm object. As another example, the energy source can be surgically applied after the annuloplasty ring has been implanted by percutaneously inserting a catheter into the patient's body and applying the energy through the catheter. For example, RF energy, light energy or thermal energy (e.g., from a heating element using resistance heating) can be transferred to the shape memory material through a catheter positioned on or near the shape memory material. Alternatively, thermal energy can be provided to the shape memory material by injecting a heated fluid through a catheter or circulating the heated fluid in a balloon through the catheter placed in close proximity to the shape memory material. As another example, the shape memory material can be coated with a photodynamic absorbing material which is activated to heat the shape memory material when illuminated by light from a laser diode or directed to the coating through fiber optic elements in a catheter. In certain such embodiments, the photodynamic absorbing material includes one or more drugs that are released when illuminated by the laser light.

In certain embodiments, a removable subcutaneous electrode or coil couples energy from a dedicated activation unit. In certain such embodiments, the removable subcutaneous electrode provides telemetry and power transmission between the system and the annuloplasty ring. The subcutaneous removable electrode allows more efficient coupling of energy to the implant with minimum or reduced power loss. In certain embodiments, the subcutaneous energy is delivered via inductive coupling.

In certain other examples, an external HIFU transducer focuses ultrasound energy onto the implanted annuloplasty ring to heat the shape memory material. In certain such embodiments, the external HIFU transducer is a handheld or portable device. The terms "HIFU," "high intensity focused ultrasound" or "focused ultrasound" as used herein are broad terms and are used at least in their ordinary sense and include, without limitation, acoustic energy within a wide range of intensities and/or frequencies. For example, HIFU includes acoustic energy focused in a region, or focal zone, having an intensity and/or frequency that is considerably less than what is currently used for ablation in medical procedures. Thus, in certain such embodiments, the focused ultrasound is not destructive to the patient's cardiac tissue. In certain embodiments, HIFU includes acoustic energy within a frequency range of approximately 0.5 MHz and approximately 30 MHz and a power density within a range of approximately 1 W/cm² and approximately 500 W/cm².

In certain examples, the annuloplasty ring comprises an ultrasound absorbing material or hydro-gel material that allows focused and rapid heating when exposed to the ultrasound energy and transfers thermal energy to the shape memory material. In certain embodiments, a HIFU probe is used with an adaptive lens to compensate for heart and respiration movement. The adaptive lens has multiple focal point adjustments. In certain embodiments, a HIFU probe with adaptive capabilities comprises a phased array or linear configuration. In certain embodiments, an external HIFU probe comprises a lens configured to be placed between a patient's ribs to improve acoustic window penetration and reduce or minimize issues and challenges regarding passing through bones. In certain embodiments, HIFU energy is synchronized with an ultrasound imaging device to allow visualization of the annuloplasty ring implant during HIFU activation. In addition, or in other embodiments, ultrasound imaging is used to non-invasively monitor the temperature of tissue surrounding the annuloplasty ring by using principles of speed of sound shift and changes to tissue thermal expansion.

In certain examples, non-invasive energy is applied to the implanted annuloplasty ring using a Magnetic Resonance Imaging (MRI) device. The shape memory material is activated by a constant magnetic field generated by the MRI device. In addition, the MRI device generates RF pulses that induce current in the annuloplasty ring and heat the shape memory material. The annuloplasty ring can include one or more coils and/or MRI energy absorbing material to increase the efficiency and directionality of the heating. Suitable energy absorbing materials for magnetic activation energy include particulates of ferromagnetic material. Suitable energy absorbing materials for RF energy include ferrite materials as well as other materials configured to absorb RF energy at resonant frequencies thereof.

In certain examples, the MRI device is used to determine the size of the implanted annuloplasty ring before, during and/or after the shape memory material is activated. In certain such examples, the MRI device generates RF pulses at a first frequency to heat the shape memory material and at a second frequency to image the implanted annuloplasty ring. Thus, the size of the annuloplasty ring can be measured without heating the ring. An MRI energy absorbing material heats sufficiently to activate the shape memory material when exposed to the first frequency and does not substantially heat when exposed to the second frequency. Other imaging techniques known in the art can also be used to determine the size of the implanted ring including, for example, ultrasound imaging, computed tomography (CT) scanning, X-ray imaging, or the like. Such imaging techniques also provide sufficient energy to activate the shape memory material.

In certain embodiments, imaging and resizing of the annuloplasty ring is performed as a separate procedure at some point after the annuloplasty ring as been surgically implanted into the patient's heart and the patient's heart, pericardium and chest have been surgically closed. However, in certain other embodiments, it is advantageous to perform the imaging after the heart and/or pericardium have been closed, but before closing the patient's chest, to check for leakage or the amount of regurgitation. If the amount of regurgitation remains excessive after the annuloplasty ring has been implanted, energy from the imaging device (or from another source as discussed herein) can be applied to the shape memory material so as to at least partially contract the annuloplasty ring and reduce regurgitation to an acceptable level. Thus, the success of the surgery can be checked and corrections can be made, if necessary, before closing the patient's chest.

In certain examples, activation of the shape memory material is synchronized with the heart beat during an imaging procedure. For example, an imaging technique can be used to focus HIFU energy onto an annuloplasty ring in a patient's body during a portion of the cardiac cycle. As the heart beats, the annuloplasty ring may move in and out of this area of focused energy. To reduce damage to the surrounding tissue, the patient's body is exposed to the HIFU energy only during portions of the cardiac cycle that focus the HIFU energy onto the cardiac ring. In certain embodiments, the energy is gated with a signal that represents the cardiac cycle such as an electrocardiogram signal. In certain such examples, the synchronization and gating is configured to allow delivery of energy to the shape memory materials at specific times during the cardiac cycle to avoid or reduce the likelihood of causing arrhythmia or fibrillation during vulnerable periods. For example, the energy can be gated so as to only expose the patient's heart to the energy during the T wave of the electrocardiogram signal.

As discussed above, shape memory materials include, for example, polymers, metals, and metal alloys including ferromagnetic alloys. Exemplary shape memory polymers that are usable for certain embodiments of the present invention are disclosed by Langer, et al. in U.S. Patent No. 6,720,402, issued April 13, 2004, U.S. Patent No. 6,388,043, issued May 14, 2002, and 6,160,084, issued December 12, 2000. Shape memory polymers respond to changes in temperature by changing to one or more permanent or memorized shapes. The shape memory polymer is heated to a temperature between approximately 38 degrees Celsius and approximately 60 degrees Celsius. The shape memory polymer is heated to a temperature in a range between approximately 40 degrees Celsius and approximately 55 degrees Celsius. The shape memory polymer has a two-way shape memory effect wherein the shape memory polymer is heated to change it to a first memorized shape and cooled to change it to a second memorized shape. The shape memory polymer can be cooled, for example, by inserting or circulating a cooled fluid through a catheter.

Shape memory polymers implanted in a patient's body can be heated non-invasively using, for example, external light energy sources such as infrared, near-infrared, ultraviolet, microwave and/or visible light sources. Preferably, the light energy is selected to increase absorption by the shape memory polymer and reduce absorption by the surrounding tissue. Thus, damage to the tissue surrounding the shape memory polymer is reduced when the shape memory polymer is heated to change its shape. In other examples, the shape memory polymer comprises gas bubbles or bubble containing liquids such as fluorocarbons and is heated by inducing a cavitation effect in the gas/liquid when exposed to HIFU energy. In other examples, the shape memory polymer may be heated using electromagnetic fields and may be coated with a material that absorbs electromagnetic fields.

Certain metal alloys have shape memory qualities and respond to changes in temperature and/or exposure to magnetic fields. Exemplary shape memory alloys that respond to changes in temperature include titanium-nickel, copper-zinc-aluminum, copper-aluminum-nickel, iron-manganese-silicon, iron-nickel-aluminum, gold-cadmium, combinations of the foregoing, and the like. In certain embodiments, the shape memory alloy comprises a biocompatible material such as a titanium-nickel alloy.

Shape memory alloys exist in two distinct solid phases called martensite and austenite. The martensite phase is relatively soft and easily deformed, whereas the austenite phase is relatively stronger and less easily deformed. For example, shape memory alloys enter the austenite phase at a relatively high temperature and the martensite phase at a relatively low temperature. Shape memory alloys begin transforming to the martensite phase at a start temperature (Mₛ) and finish transforming to the martensite phase at a finish temperature (M_{f}). Similarly, such shape memory alloys begin transforming to the austenite phase at a start temperature (Aₛ) and finish transforming to the austenite phase at a finish temperature (A_{f}). Both transformations have a hysteresis. Thus, the Mₛ temperature and the A_{f} temperature are not coincident with each other, and the M_{f} temperature and the Aₛ temperature are not coincident with each other.

In certain embodiments, the shape memory alloy is processed to form a memorized shape in the austenite phase in the form of a ring or partial ring. The shape memory alloy is then cooled below the M_{f} temperature to enter the martensite phase and deformed into a larger or smaller ring. For example, in certain embodiments, the shape memory alloy is formed into a ring or partial ring that is larger than the memorized shape but still small enough to improve leaflet coaptation and reduce regurgitation in a heart valve upon being attached to the heart valve annulus. In certain such embodiments, the shape memory alloy is sufficiently malleable in the martensite phase to allow a user such as a physician to adjust the circumference of the ring in the martensite phase by hand to achieve a desired fit for a particular heart valve annulus. After the ring is attached to the heart valve annulus, the circumference of the ring can be adjusted non-invasively by heating the shape memory alloy to an activation temperature (e.g., temperatures ranging from the Aₛ temperature to the A_{f} temperature).

Thereafter, when the shape memory alloy is exposed to a temperature elevation and transformed to the austenite phase, the alloy changes in shape from the deformed shape to the memorized shape. Activation temperatures at which the shape memory alloy causes the shape of the annuloplasty ring to change shape can be selected and built into the annuloplasty ring such that collateral damage is reduced or eliminated in tissue adjacent the annuloplasty ring during the activation process. Exemplary A_{f} temperatures for suitable shape memory alloys range between approximately 45 degrees Celsius and approximately 70 degrees Celsius. Furthermore, exemplary Mₛ temperatures range between approximately 10 degrees Celsius and approximately 20 degrees Celsius, and exemplary M_{f} temperatures range between approximately -1 degrees Celsius and approximately 15 degrees Celsius. The size of the annuloplasty ring can be changed all at once or incrementally in small steps at different times in order to achieve the adjustment necessary to produce the desired clinical result.

Certain shape memory alloys may further include a rhombohedral phase, having a rhombohedral start temperature (Rₛ) and a rhombohedral finish temperature (R_{f}), that exists between the austenite and martensite phases. An example of such a shape memory alloy is a NiTi alloy, which is commercially available from Memry Corporation (Bethel, Connecticut). In certain embodiments, an exemplary Rₛ temperature range is between approximately 30 degrees Celsius and approximately 50 degrees Celsius, and an exemplary R_{f} temperature range is between approximately 20 degrees Celsius and approximately 35 degrees Celsius. One benefit of using a shape memory material having a rhombohedral phase is that in the rhomobohedral phase the shape memory material may experience a partial physical distortion, as compared to the generally rigid structure of the austenite phase and the generally deformable structure of the martensite phase.

Certain shape memory alloys exhibit a ferromagnetic shape memory effect wherein the shape memory alloy transforms from the martensite phase to the austenite phase when exposed to an external magnetic field. The term "ferromagnetic" as used herein is a broad term and is used in its ordinary sense and includes, without limitation, any material that easily magnetizes, such as a material having atoms that orient their electron spins to conform to an external magnetic field. Ferromagnetic materials include permanent magnets, which can be magnetized through a variety of modes, and materials, such as metals, that are attracted to permanent magnets. Ferromagnetic materials also include electromagnetic materials that are capable of being activated by an electromagnetic transmitter, such as one located outside the heart. Furthermore, ferromagnetic materials may include one or more polymer-bonded magnets, wherein magnetic particles are bound within a polymer matrix, such as a biocompatible polymer. The magnetic materials can comprise isotropic and/or anisotropic materials, such as for example NdFeB (Neodynium Iron Boron), SmCo (Samarium Cobalt), ferrite and/or AlNiCo (Aluminum Nickel Cobalt) particles.

Thus, an annuloplasty ring comprising a ferromagnetic shape memory alloy can be implanted in a first configuration having a first shape and later changed to a second configuration having a second (e.g., memorized) shape without heating the shape memory material above the Aₛ temperature. Advantageously, nearby healthy tissue is not exposed to high temperatures that could damage the tissue. Further, since the ferromagnetic shape memory alloy does not need to be heated, the size of the annuloplasty ring can be adjusted more quickly and more uniformly than by heat activation.

Exemplary ferromagnetic shape memory alloys include Fe-C, Fe-Pd, Fe-Mn-Si, Co-Mn, Fe-Co-Ni-Ti, Ni-Mn-Ga, Ni₂MnGa, Co-Ni-Al, and the like. Certain of these shape memory materials may also change shape in response to changes in temperature. Thus, the shape of such materials can be adjusted by exposure to a magnetic field, by changing the temperature of the material, or both.

In certain embodiments, combinations of different shape memory materials are used. For example, annuloplasty rings according to certain embodiments comprise a combination of shape memory polymer and shape memory alloy (e.g., NiTi). In certain such embodiments, an annuloplasty ring comprises a shape memory polymer tube and a shape memory alloy (e.g., NiTi) disposed within the tube. Such embodiments are flexible and allow the size and shape of the shape memory to be further reduced without impacting fatigue properties. In addition, or in other embodiments, shape memory polymers are used with shape memory alloys to create a bi-directional (e.g., capable of expanding and contracting) annuloplasty ring. Bi-directional annuloplasty rings can be created with a wide variety of shape memory material combinations having different characteristics.

In the following description, reference is made to the accompanying drawings, which form a part hereof, and which show, by way of illustration, specific embodiments or processes in which the invention may be practiced. Where possible, the same reference numbers are used throughout the drawings to refer to the same or like components. In some instances, numerous specific details are set forth in order to provide a thorough understanding of the present disclosure. The present disclosure, however, may be practiced without the specific details or with certain alternative equivalent components and methods to those described herein. In other instances, well-known components and methods have not been described in detail so as not to unnecessarily obscure aspects of the present disclosure.

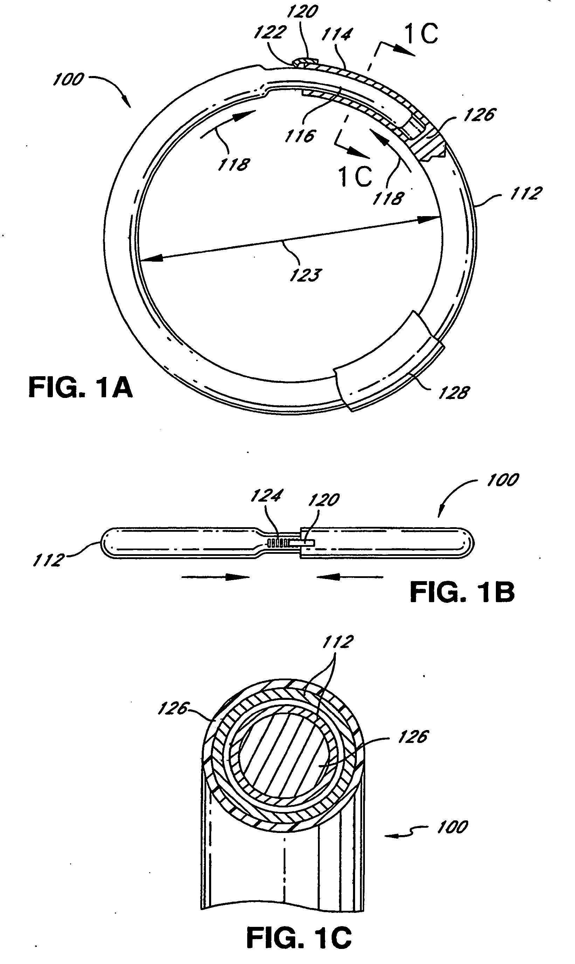

FIGS. 1A-1C illustrate an adjustable annuloplasty ring 100 not part of the invention that can be adjusted in vivo after implantation into a patient's body. The annuloplasty ring 100 has a substantially annular configuration and comprises a tubular body member 112 that folds back upon itself in a substantial circle having a nominal diameter as indicated by arrow 123. The tubular body member 112 comprises a receptacle end 114 and an insert end 116. The insert end 116 of the tubular member 112 is reduced in outer diameter or transverse dimension as compared to the receptacle end 114. As used herein, "dimension" is a broad term having its ordinary and customary meaning and includes a size or distance from a first point to a second point along a line or arc. For example, a dimension may be a circumference, diameter, radius, arc length, or the like. As another example, a dimension may be a distance between an anterior portion and a posterior portion of an annulus.

The receptacle end accepts the insert end 116 of the tubular member 112 to complete the ring-like structure of the annuloplasty ring 100. The insert end 116 slides freely within the receptacle end 114 of the annuloplasty ring 100 which allows contraction of the overall circumference of the ring 100 as the insert end 116 enters the receptacle end 114 as shown by arrows 118 in FIG. 1A. In certain embodiments, the nominal diameter or transverse dimension 123 of the annuloplasty ring 100 can be adjusted from approximately 25 mm to approximately 38 mm. However, an artisan will recognize from the disclosure herein that the diameter or transverse dimension 123 of the annuloplasty ring 100 can be adjusted to other sizes depending on the particular application. Indeed, the diameter or transverse dimension 123 of the annuloplasty ring 100 can be configured to reinforce body structures substantially smaller than 25 mm and substantially larger than 38 mm.

An artisan will recognize from the disclosure herein that in other embodiments the insert end 116 can couple with the receptacle end 114 without being inserted in the receptacle end 114. For example, the insert end 116 can overlap the receptacle end 114 such that it slides adjacent thereto. In other embodiments, for example, the ends 114, 116 may grooved to guide the movement of the adjacent ends 114, 116 relative to one another. Other embodiments within the scope of the invention will occur to those skilled in the art.

The annuloplasty ring 100 also comprises a suturable material 128, shown partially cut away in FIG. 1A, and not shown in FIGS. 1B and 1C for clarity. The suturable material 128 is disposed about the tubular member 112 to facilitate surgical implantation of the annuloplasty ring 100 in a body structure, such as about a heart valve annulus. In certain examples, the suturable material 128 comprises a suitable biocompatible material such as Dacron@, woven velour, polyurethane, polytetrafluoroethylene (PTFE), heparin-coated fabric, or the like. In other embodiments, the suturable material 128 comprises a biological material such as bovine or equine pericardium, homograft, patient graft, or cell-seeded tissue. The suturable material 128 may be disposed about the entire circumference of the tubular member 112, or selected portions thereof. For example, in certain embodiments, the suturable material 128 is disposed so as to enclose substantially the entire tubular member 112 except at the narrowed insert end 116 that slides into the receptacle end 118 of the tubular member 112.

As shown in FIGS. 1A and 1B, in certain examples, the annuloplasty ring 100 also comprises a ratchet member 120 secured to the receptacle end 114 of the tubular member 112. The ratchet member 120 comprises a pawl 122 configured to engage transverse slots 124 (shown in FIG. 1B) on the insert end 116 of the tubular member 112. The pawl 122 of the ratchet member 120 engages the slots 124 in such a way as to allow contraction of the circumference of the annuloplasty ring 100 and prevent or reduce circumferential expansion of the annuloplasty ring 100. Thus, the ratchet reduces unwanted circumferential expansion of the annuloplasty ring 100 after implantation due, for example, to dynamic forces on the annuloplasty ring 100 from the heart tissue during systolic contraction of the heart.

In certain examples, the tubular member 112 comprises a rigid material such as stainless steel, titanium, or the like, or a flexible material such as silicon rubber, Dacron®, or the like. In certain such examples, after implantation into a patient's body, the circumference of the annuloplasty ring 100 is adjusted in vivo by inserting a catheter (not shown) into the body and pulling a wire (not shown) attached to the tubular member 112 through the catheter to manually slide the insert end 116 of the tubular member 112 into the receptacle end 114 of the tubular member 112. As the insert end 116 slides into the receptacle end 114, the pawl 122 of the ratchet member 120 engages the slots 124 on the insert end 116 to hold the insert end 116 in the receptacle end 114. Thus, for example, as the size of a heart valve annulus reduces after implantation of the annuloplasty ring 100, the size of the annuloplasty ring 100 can also be reduced to provide an appropriate amount of reinforcement to the heart valve.

In certain other examples, the tubular member 112 comprises a shape memory material that is responsive to changes in temperature and/or exposure to a magnetic field. As discussed above, the shape memory material may include shape memory polymers (e.g., polylactic acid (PLA), polyglycolic acid (PGA)) and/or shape memory alloys (e.g., nickel-titanium) including ferromagnetic shape memory alloys (e.g., Fe-C, Fe-Pd, Fe-Mn-Si, Co-Mn, Fe-Co-Ni-Ti, Ni-Mn-Ga, Ni₂MnGa, Co-Ni-Al). In certain such examples, the annuloplasty ring 100 is adjusted in vivo by applying an energy source such as radio frequency energy, X-ray energy, microwave energy, ultrasonic energy such as high intensity focused ultrasound (HIFU) energy, light energy, electric field energy, magnetic field energy, combinations of the foregoing, or the like. Preferably, the energy source is applied in a non-invasive manner from outside the body. For example, as discussed above, a magnetic field and/or RF pulses can be applied to the annuloplasty ring 100 within a patient's body with an apparatus external to the patient's body such as is commonly used for magnetic resonance imaging (MRI). However, in other examples, the energy source may be applied surgically such as by inserting a catheter into the body and applying the energy through the catheter.

In certain examples, the tubular body member 112 comprises a shape memory material that responds to the application of temperature that differs from a nominal ambient temperature, such as the nominal body temperature of 37 degrees Celsius for humans. The tubular member 112 is configured to respond by starting to contract upon heating the tubular member 112 above the Aₛ temperature of the shape memory material. In certain such examples, the annuloplasty ring 100 has an initial diameter or transverse dimension 123 of approximately 30 mm, and contracts or shrinks to a transverse dimension 123 of approximately 23 mm to approximately 28 mm, or any increment between those values. This produces a contraction percentage in a range between approximately 6 percent and approximately 23 percent, where the percentage of contraction is defined as a ratio of the difference between the starting diameter and finish diameter divided by the starting diameter.

The activation temperatures (e.g., temperatures ranging from the Aₛ temperature to the A_{f} temperature) at which the tubular member 112 contracts to a reduced circumference may be selected and built into the annuloplasty ring 100 such that collateral damage is reduced or eliminated in tissue adjacent the annuloplasty ring 100 during the activation process. Exemplary A_{f} temperatures for the shape memory material of the tubular member 112 at which substantially maximum contraction occurs are in a range between approximately 38 degrees Celsius and approximately 75 degrees Celsius. In certain examples, the A_{f} temperature is in a range between approximately 39 degrees Celsius and approximately 75 degrees Celsius. For some examples that include shape memory polymers for the tubular member 112, activation temperatures at which the glass transition of the material or substantially maximum contraction occur range between approximately 38 degrees Celsius and approximately 60 degrees Celsius. In other such embodiments, the activation temperature is in a range between approximately 40 degrees Celsius and approximately 59 degrees Celsius.

In certain examples, the tubular member 112 is shape set in the austenite phase to a remembered configuration during the manufacturing of the tubular member 112 such that the remembered configuration is that of a relatively small circumferential value with the insert end 116 fully inserted into the receptacle end 114. After cooling the tubular member 112 below the M_{f} temperature, the tubular member 112 is manually deformed to a larger circumferential value with the insert end 116 only partially inserted into the receptacle end 114 to achieve a desired starting nominal circumference for the annuloplasty ring 100. In certain such examples, the tubular member 112 is sufficiently malleable in the martensite phase to allow a user such as a physician to adjust the circumferential value by hand to achieve a desired fit with the heart valve annulus. In certain examples, the starting nominal circumference for the annuloplasty ring 100 is configured to improve leaflet coaptation and reduce regurgitation in a heart valve.

After implantation, the annuloplasty ring 100 is preferably activated non-invasively by the application of energy to the patient's body to heat the tubular member 112. In certain examples, an MRI device is used as discussed above to heat the tubular member 112, which then causes the shape memory material of the tubular member 112 to transform to the austenite phase and remember its contracted configuration. Thus, the circumference of the annuloplasty ring 100 is reduced in vivo without the need for surgical intervention. Standard techniques for focusing the magnetic field from the MRI device onto the annuloplasty ring 100 may be used. For example, a conductive coil can be wrapped around the patient in an area corresponding to the annuloplasty ring 100. In other examples, the shape memory material is activated by exposing it other sources of energy, as discussed above.

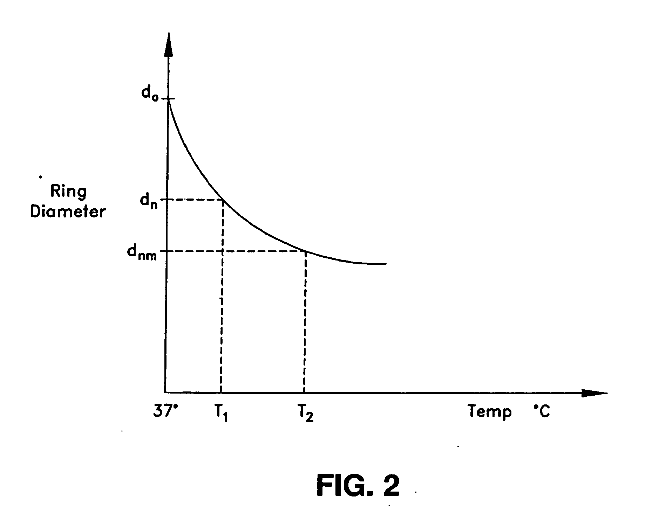

The circumference reduction process, either non-invasively or through a catheter, can be carried out all at once or incrementally in small steps at different times in order to achieve the adjustment necessary to produce the desired clinical result. If heating energy is applied such that the temperature of the tubular member 112 does not reach the A_{f} temperature for substantially maximum transition contraction, partial shape memory transformation and contraction may occur. FIG. 2 graphically illustrates the relationship between the temperature of the tubular member 112 and the diameter or transverse dimension 123 of the annuloplasty ring 100 according to certain embodiments. At body temperature of approximately 37 degrees Celsius, the diameter of the tubular member 112 has a first diameter do. The shape memory material is then increased to a first raised temperature T₁. In response, the diameter of the tubular member 112 reduces to a second diameter dₙ. The diameter of the tubular member 112 can then be reduced to a third diameter dₙₘ by raising the temperature to a second temperature T₂.

As graphically illustrated in FIG. 2, in certain examples, the change in diameter from do to dₙₘ is substantially continuous as the temperature is increased from body temperature to T₂. For example, in certain examples a magnetic field of about 2.5 Tesla to about 3.0 Tesla is used to raise the temperature of the tubular member 112 above the A_{f} temperature to complete the austenite phase and return the tubular member 112 to the remembered configuration with the insert end 116 fully inserted into the receptacle end 114. However, a lower magnetic field (e.g., 0.5 Tesla) can initially be applied and increased (e.g., in 0.5 Tesla increments) until the desired level of heating and desired contraction of the annuloplasty ring 100 is achieved. In other examples, the tubular member 112 comprises a plurality of shape memory materials with different activation temperatures and the diameter of the tubular member 112 is reduced in steps as the temperature increases.

Whether the shape change is continuous or stepped, the diameter or transverse dimension 123 of the ring 100 can be assessed or monitored during the contraction process to determine the amount of contraction by use of MRI imaging, ultrasound imaging, computed tomography (CT), X-ray or the like. If magnetic energy is being used to activate contraction of the ring 100, for example, MRI imaging techniques can be used that produce a field strength that is lower than that required for activation of the annuloplasty ring 100.

In certain examples, the tubular member 112 comprises an energy absorption enhancement material 126. As shown in FIGS. 1A and 1C, the energy absorption enhancement material 126 may be disposed within an inner chamber of the tubular member 112. As shown in FIG. 1C (and not shown in FIG. 1A for clarity), the energy absorption enhancement material 126 may also be coated on the outside of the tubular member 112 to enhance energy absorption by the tubular member 112. For examples that use energy absorption enhancement material 126 for enhanced absorption, it may be desirable for the energy absorption enhancement material 126, a carrier material (not shown) surrounding the energy absorption enhancement material 126, if there is one, or both to be thermally conductive. Thus, thermal energy from the energy absorption enhancement material 126 is efficiently transferred to the shape memory material of the annuloplasty ring 100, such as the tubular member 112.

As discussed above, the energy absorption enhancement material 126 may include a material or compound that selectively absorbs a desired heating energy and efficiently converts the non-invasive heating energy to heat which is then transferred by thermal conduction to the tubular member 112. The energy absorption enhancement material 126 allows the tubular member 112 to be actuated and adjusted by the non-invasive application of lower levels of energy and also allows for the use of non-conducting materials, such as shape memory polymers, for the tubular member 112. For some examples, magnetic flux ranging between about 2.5 Tesla and about 3.0 Tesla may be used for activation. In certain embodiments, magnetic flux ranging between 2.0 Tesla and 3.5 Tesla may be used for activation. By allowing the use of lower energy levels, the energy absorption enhancement material 126 also reduces thermal damage to nearby tissue. Suitable energy absorption enhancement materials 126 are discussed above.

In certain examples, a circumferential contraction cycle can be reversed to induce an expansion of the annuloplasty ring 100. Some shape memory alloys, such as NiTi or the like, respond to the application of a temperature below the nominal ambient temperature. After a circumferential contraction cycle has been performed, the tubular member 112 is cooled below the Mₛ temperature to start expanding the annuloplasty ring 100. The tubular member 112 can also be cooled below the M_{f} temperature to finish the transformation to the martensite phase and reverse the contraction cycle. As discussed above, certain polymers also exhibit a two-way shape memory effect and can be used to both expand and contract the annuloplasty ring 100 through heating and cooling processes. Cooling can be achieved, for example, by inserting a cool liquid onto or into the annuloplasty ring 100 through a catheter, or by cycling a cool liquid or gas through a catheter placed near the annuloplasty ring 100. Exemplary temperatures for a NiTi embodiment for cooling and reversing a contraction cycle range between approximately 20 degrees Celsius and approximately 30 degrees Celsius.

In certain examples, external stresses are applied to the tubular member 112 during cooling to expand the annuloplasty ring 100. In certain such examples, one or more biasing elements (not shown) are operatively coupled to the tubular member 112 so as to exert a circumferentially expanding force thereon. For example, in certain examples a biasing element such as a spring (not shown) is disposed in the receptacle end 114 of the tubular member 112 so as to push the insert end 16 at least partially out of the receptacle end 114 during cooling. In such embodiments, the tubular member 112 does not include the ratchet member 120 such that the insert end 116 can slide freely into or out of the receptacle end 114.

In certain examples, the tubular member comprises ferromagnetic shape memory material, as discussed above. In such examples, the diameter of the tubular member 112 can be changed by exposing the tubular member 112 to a magnetic field. Advantageously, nearby healthy tissue is not exposed to high temperatures that could damage the tissue. Further, since the shape memory material does not need to be heated, the size of the tubular member 112 can be adjusted more quickly and more uniformly than by heat activation.

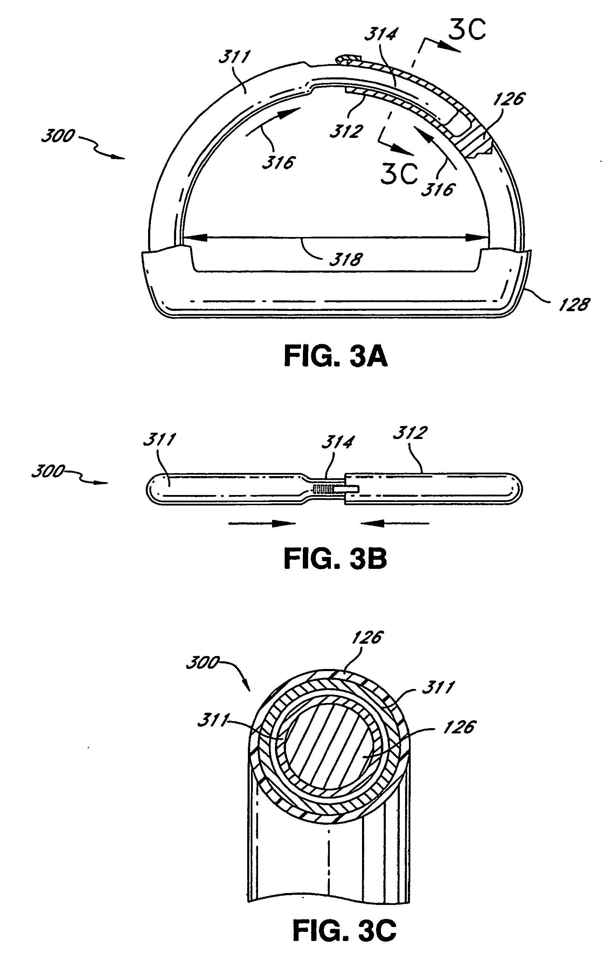

FIGS. 3A-3C illustrate an example of an adjustable annuloplasty ring 300 that is similar to the annuloplasty ring 100 discussed above, but having a D-shaped configuration instead of a circular configuration. The annuloplasty ring 300 comprises a tubular body member 311 having a receptacle end 312 and an insert end 314 sized and configured to slide freely in the hollow receptacle end 312 in an axial direction which allows the annuloplasty ring 300 to constrict upon activation to a lesser circumference or transverse dimension as indicated by arrows 316. The annuloplasty ring 300 has a major transverse dimension indicated by arrow 318 that is reduced upon activation of the annuloplasty ring 300. The major transverse dimension indicated by arrow 318 can be the same as or similar to the transverse dimension indicated by arrow 123 discussed above. In certain examples, the features, dimensions and materials of the annuloplasty ring 300 are the same as or similar to the features, dimensions and materials of annuloplasty ring 100 discussed above. The D-shaped configuration of ring 32 allows a proper fit of the ring 32 with the morphology of some particular heart valves.

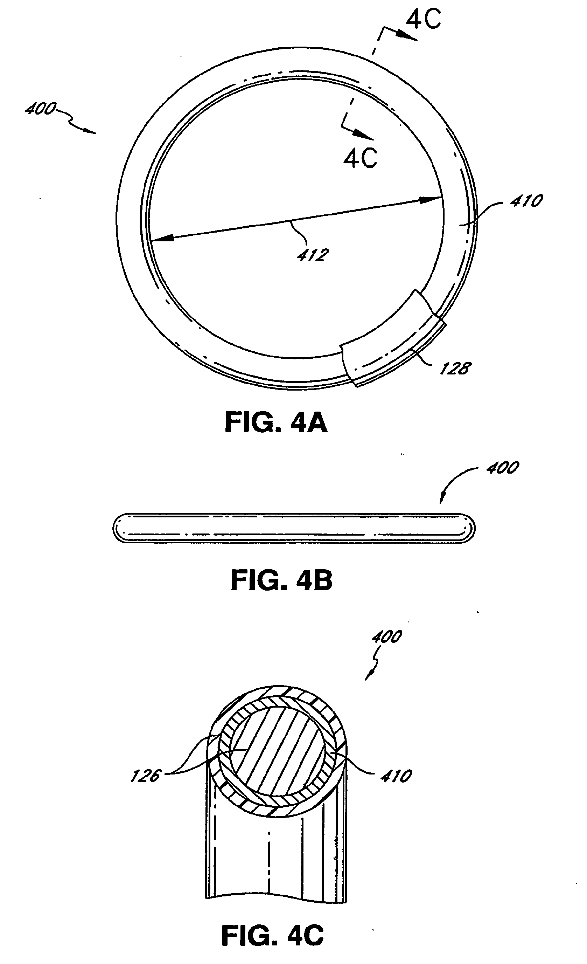

FIGS. 4A-4C show an example not according to the invention of an annuloplasty ring 400 that includes a continuous tubular member 410 surrounded by a suturable material 128. The tubular member 410 has a substantially circular transverse cross section, as shown in FIG. 4C, and has an absorption enhancing material 126 disposed within an inner chamber of the tubular member 410. In certain examples, the absorption enhancing material 126 is also disposed on the outer surface of the tubular member 410. The tubular member 410 may be made from a shape memory material such as a shape memory polymer or a shape memory alloy including a ferromagnetic shape memory alloy, as discussed above.

For examples of the annuloplasty ring 400 with a tubular member 410 made from a continuous piece of shape memory alloy (e.g., NiTi alloy) or shape memory polymer, the annuloplasty ring 400 can be activated by the surgical and/or non-invasive application of heating energy by the methods discussed above with regard to other examples. For examples of the annuloplasty ring 400 with a tubular member 410 made from a continuous piece of ferromagnetic shape memory alloy, the annuloplasty ring 400 can be activated by the non-invasive application of a suitable magnetic field. The annuloplasty ring 400 has a nominal inner diameter or transverse dimension indicated by arrow 412 in FIG. 4A that is set during manufacture of the ring 400. In certain examples, the annuloplasty ring 400 is sufficiently malleable when it is implanted into a patient's body that it can be adjusted by hand to be fitted to a particular heart valve annulus.

In certain examples, upon activating the tubular member 410 by the application of energy, the tubular member 410 remembers and assumes a configuration wherein the transverse dimension is less than the nominal transverse dimension 412. A contraction in a range between approximately 6 percent to approximately 23 percent may be desirable in some embodiments which have continuous hoops of shape memory tubular members 410. In certain examples, the tubular member 410 comprises a shape memory NiTi alloy having an inner transverse dimension in a range between approximately 25 mm and approximately 38 mm. In certain such examples, the tubular member 410 can contract or shrink in a range between approximately 6 percent and approximately 23 percent, where the percentage of contraction is defined as a ratio of the difference between the starting diameter and finish diameter divided by the starting diameter. In certain embodiments, the annuloplasty ring 400 has a nominal inner transverse dimension 412 of approximately 30 mm and an inner transverse dimension in a range between approximately 23 mm and approximately 128 mm in a fully contracted state.

As discussed above in relation to FIG. 2, in certain examples, the inner transverse dimension 412 of certain embodiments can be altered as a function of the temperature of the tubular member 410. As also discussed above, in certain such examples, the progress of the size change can be measured or monitored in real-time conventional imaging techniques. Energy from conventional imaging devices can also be used to activate the shape memory material and change the inner transverse dimension 412 of the tubular member 410. In certain examples, the features, dimensions and materials of the annuloplasty ring 400 are the same as or similar to the features, dimensions and materials of the annuloplasty ring 100 discussed above. For example, in certain examples, the tubular member 410 comprises a shape memory material that exhibits a two-way shape memory effect when heated and cooled. Thus, the annuloplasty ring 400, in certain such examples, can be contracted and expanded.

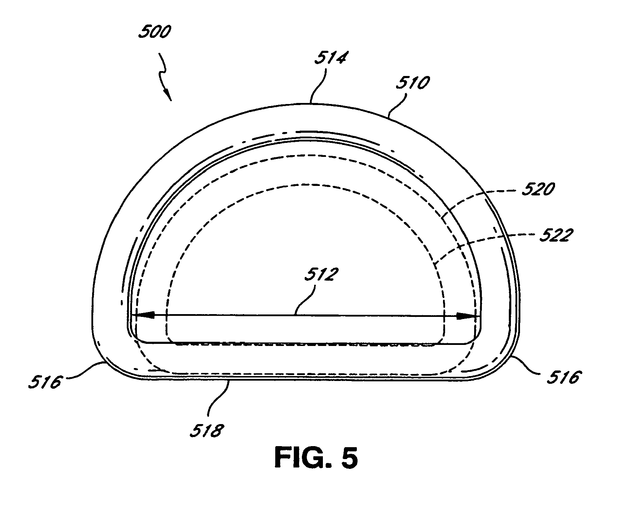

FIG. 5 illustrates a top view of an annuloplasty ring 500 having a D-shaped configuration not according to the invention. The annuloplasty ring 500 includes a continuous tubular member 510 comprising a shape memory material that has a nominal inner transverse dimension indicated by arrow 512 that may contract or shrink upon the activation of the shape memory material by surgically or non-invasive applying energy thereto, as discussed above. The tubular member 510 may comprise a homogeneous shape memory material, such as a shape memory polymer or a shape memory alloy including, for example, a ferromagnetic shape memory alloy.

Alternatively, the tubular member 510 may comprise two or more sections or zones of shape memory material having different temperature response curves. The shape memory response zones may be configured in order to achieve a desired configuration of the annuloplasty ring 500 as a whole when in a contracted state, either fully contracted or partially contracted. For example, the tubular member 510 may have a first zone or section 514 that includes the arched portion of the tubular member that terminates at or near the corners 516 and a second zone or section 518 that includes the substantially straight portion of the tubular member 510 disposed directly between the corners 516.

The annuloplasty ring 500 is shown in a contracted state in FIG. 5 as indicated by the dashed lines 520, 522, which represent contracted states of certain examples wherein both the first section 514 and second section 518 of the tubular member 510 have contracted axially. A suturable material (not shown), such as the suturable material 128 shown in FIG. 1, may be disposed about the tubular member 510 and the tubular member 510 may comprise or be coated with an energy absorption enhancement material 126, as discussed above. In certain examples, the features, dimensions and materials of the annuloplasty ring 500 are the same as or similar to the features, dimensions and materials of the annuloplasty ring 100 discussed above.

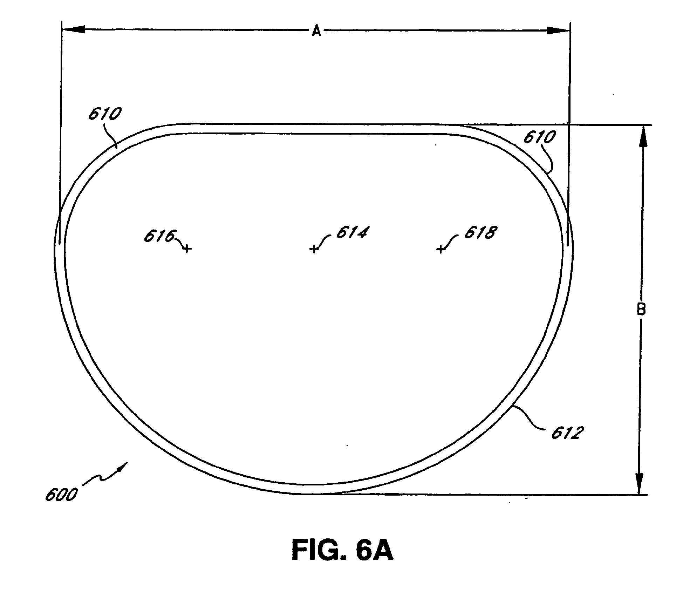

FIG. 6A is a schematic diagram of a top view of a substantially D-shaped wire 600 comprising a shape memory material not according to the invention. The term "wire" is a broad term having its normal and customary meaning and includes, for example, mesh, flat, round, rod-shaped, or band-shaped members. Suitable shape memory materials include shape memory polymers or shape memory alloys including, for example, ferromagnetic shape memory alloys, as discussed above. The wire 600 comprises a substantially linear portion 608, two corner portions 610, and a substantially semi-circular portion 612.

For purposes of discussion, the wire 600 is shown relative to a first reference point 614, a second reference point 616 and a third reference point 618. The radius of the substantially semi-circular portion 612 is defined with respect to the first reference point 614 and the corner portions 610 are respectively defined with respect to the second reference point 616 and the third reference point 618. Also for purposes of discussion, FIG. 6A shows a first transverse dimension A, a second transverse dimension B.

In certain examples, the first transverse dimension A is in a range between approximately 20.0 mm and approximately 40.0 mm, the second transverse dimension B is in a range between approximately 10.0 mm and approximately 25.0 mm. In certain such examples, the wire 600 comprises a rod having a diameter in a range between approximately 0.45 mm and approximately 0.55 mm, the radius of each corner portion 610 is in a range between approximately 5.8 mm and 7.2 mm, and the radius of the substantially semi-circular portion 612 is in a range between approximately 11.5 mm and approximately 14.0 mm. In certain other such examples, the wire 600 comprises a rod having a diameter in a range between approximately 0.90 mm and approximately 1.10 mm, the radius of each corner portion 610 is in a range between approximately 6.1 mm and 7.4 mm, and the radius of the substantially semi-circular portion 612 is in a range between approximately 11.7 mm and approximately 14.3 mm.

In certain other examples, the first transverse dimension A is in a range between approximately 26.1 mm and approximately 31.9 mm, the second transverse dimension B is in a range between approximately 20.3 mm and approximately 24.9 mm. In certain such examples, the wire 600 comprises a rod having a diameter in a range between approximately 0.4 mm and approximately 0.6 mm, the radius of each corner portion 610 is in a range between approximately 6.7 mm and 8.3 mm, and the radius of the substantially semi-circular portion 612 is in a range between approximately 13.3 mm and approximately 16.2 mm. In certain other such examples, the wire 600 comprises a rod having a diameter in a range between approximately 0.90 mm and approximately 1.10 mm, the radius of each corner portion 610 is in a range between approximately 6.9 mm and 8.5 mm, and the radius of the substantially semi-circular portion 612 is in a range between approximately 13.5 mm and approximately 16.5 mm.

In certain examples, the wire 600 comprises a NiTi alloy configured to transition to its austenite phase when heated so as to transform to a memorized shape, as discussed above. In certain such examples, the first transverse dimension A of the wire 600 is configured to be reduced by approximately 10% to 25% when transitioning to the austenite phase. In certain such examples, the austenite start temperature As is in a range between approximately 33 degrees Celsius and approximately 43 degrees Celsius, the austenite finish temperature A_{f} is in a range between approximately 45 degrees Celsius and approximately 55 degrees Celsius, the martensite start temperature Mₛ is less than approximately 30 degrees Celsius, and the martensite finish temperature M_{f} is greater than approximately 20 degrees Celsius. In other examples, the austenite finish temperature A_{f} is in a range between approximately 48.75 degrees Celsius and approximately 51.25 degrees Celsius. Other examples can include other start and finish temperatures for martensite, rhombohedral and austenite phases as described herein.

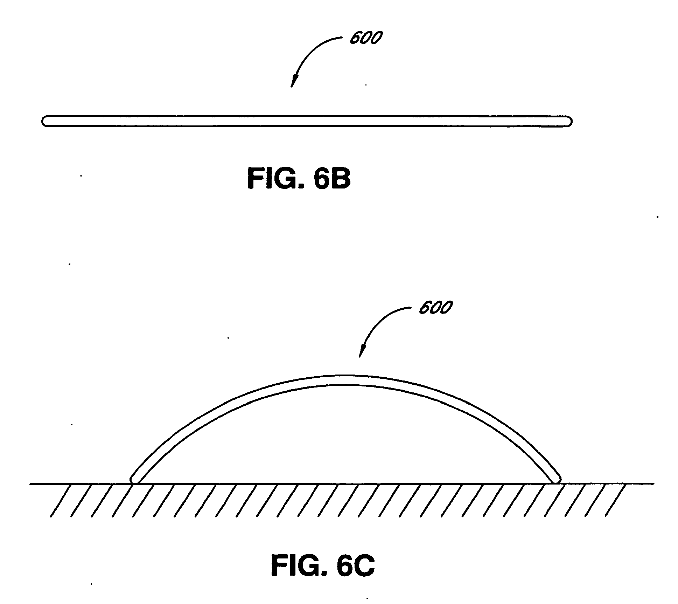

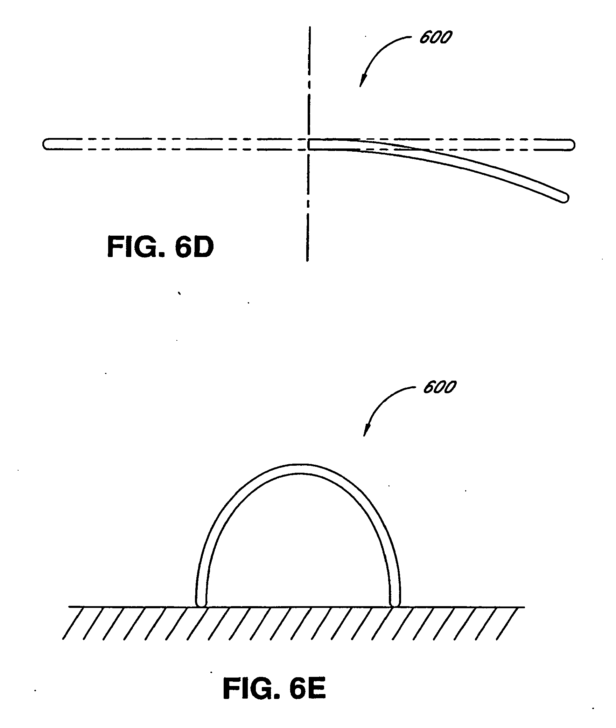

FIGS. 6B-6E are schematic diagrams of side views of the shape memory wire 600 of FIG. 6A. In addition to expanding and/or contracting the first transverse dimension A and/or the second transverse dimension B when transitioning to the austenite phase, in certain embodiments the shape memory wire 600 is configured to change shape in a third dimension perpendicular to the first transverse dimension A and the second transverse dimension B. For example, the shape memory wire 600 is substantially planar or flat in the third dimension, as shown in FIG. 6B, when implanted into a patient's body. Then, after implantation, the shape memory wire 600 is activated such that it expands or contracts in the first transverse dimension A and/or the second transverse dimension B and flexes or bows in the third dimension such that it is no longer planar, as shown in FIG. 6C. Such bowing may be symmetrical as shown in FIG. 6C or asymmetrical as shown in FIG. 6D to accommodate the natural shape of the annulus.

In certain examples, the shape memory wire 600 is configured to bow in the third dimension a distance in a range between approximately 2 millimeters and approximately 10 millimeters. In certain examples, the shape memory wire 600 is implanted to bow towards the atrium when implanted around a cardiac valve annulus to accommodate the natural shape of the annulus. In other examples, the shape memory wire 600 is configured to bow towards the ventricle when implanted around a cardiac valve to accommodate the natural shape of the annulus.

In certain examples, the shape memory wire 600 is bowed in the third dimension, as shown in FIG. 6C, when implanted into the patient's body. Then, after implantation, the shape memory wire 600 is activated such that it expands or contracts in the first transverse dimension A and/or the second transverse dimension B and further flexes or bows in the third dimension, as shown in FIG. 6E. In certain other examples, the shape memory wire 600 is bowed in the third dimension, as shown in FIG. 6C, when implanted into the patient's body. Then, after implantation, the shape memory wire 600 is activated such that it expands or contracts in the first transverse dimension A and/or the second transverse dimension B and changes shape in the third dimension so as to become substantially flat, as shown in FIG. 6B. An artisan will recognize from the disclosure herein that other annuloplasty rings disclosed herein can also be configured to bow or change shape in a third dimension so as to accommodate or further reinforce a valve annulus.

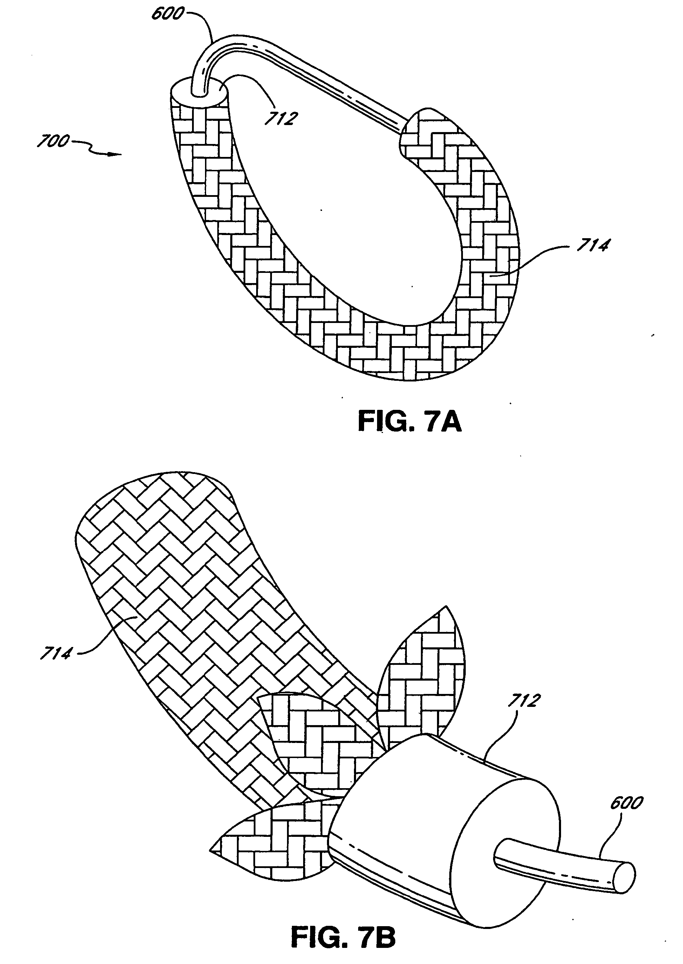

FIG. 7A is a perspective view illustrating portions of an annuloplasty ring 700 comprising the wire 600 shown in FIG. 6A. The wire 600 is covered by a flexible material 712 such as silicone rubber and a suturable material 714 such as woven polyester cloth, Dacron@, woven velour, polyurethane, polytetrafluoroethylene (PTFE), heparin-coated fabric, or other biocompatible material. The suturable material 714 comprises a biological material such as bovine or equine pericardium, homograft, patient graft, or cell-seeded tissue. For illustrative purposes, portions of the flexible material 712 and the suturable material 714 are not shown in FIG. 7A to show the wire 600. However, in certain examples, the flexible material 712 and the suturable material 714 are continuous and cover substantially the entire wire 600. Although not shown, in certain examples, the wire 600 is coated with an energy absorption enhancement material, as discussed above.

FIG. 7B is an enlarged perspective view of a portion of the annuloplasty ring 700 shown in FIG. 7A. For illustrative purposes, portions of the flexible material 712 are not shown to expose the wire 600 and portions of the suturable material 714 are shown peeled back to expose the flexible material 712. In certain embodiments, the diameter of the flexible material 712 is in a range between approximately 0.10 inches and approximately 0.15 inches. FIG. 7B shows the wire 600 substantially centered within the circumference of the flexible material 712. However, in certain examples, the wire 600 is offset within the circumference of the flexible material 712 to allow more space for sutures.

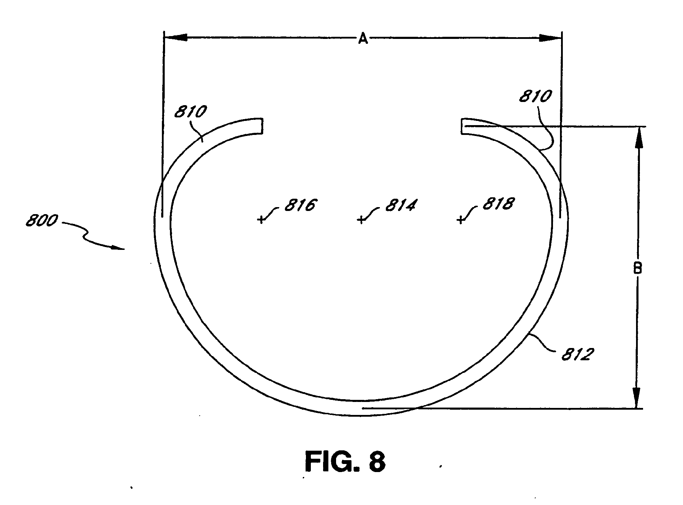

FIG. 8 is a schematic diagram of a substantially C-shaped wire 800 comprising a shape memory material not according to the invention. Suitable shape memory materials include shape memory polymers or shape memory alloys including, for example, ferromagnetic shape memory alloys, as discussed above. The wire 800 comprises two corner portions 810, and a substantially semi-circular portion 812.

For purposes of discussion, the wire 800 is shown relative to a first reference point 814, a second reference point 816 and a third reference point 818. The radius of the substantially semi-circular portion 812 is defined with respect to the first reference point 814 and the corner portions 810 are respectively defined with respect to the second reference point 816 and the third reference point 818. Also for purposes of discussion, FIG. 8 shows a first transverse dimension A and a second transverse dimension B. In certain examples, the wire 800 comprises a rod having a diameter and dimensions A and B as discussed above in relation to FIG. 6A.

In certain examples, the wire 800 comprises a NiTi alloy configured to transition to its austenite phase when heated so as to transform to a memorized shape, as discussed above. In certain such examples, the first transverse dimension A of the wire 800 is configured to be reduced by approximately 10% to 25% when transitioning to the austenite phase. In certain such embodiments, the austenite start temperature Aₛ is in a range between approximately 33 degrees Celsius and approximately 43 degrees Celsius, the austenite finish temperature A_{f} is in a range between approximately 45 degrees Celsius and approximately 55 degrees Celsius, the martensite start temperature Mₛ is less than approximately 30 degrees Celsius, and the martensite finish temperature M_{f} is greater than approximately 20 degrees Celsius. In other examples, the austenite finish temperature A_{f} is in a range between approximately 48.75 degrees Celsius and approximately 51.25 degrees Celsius.

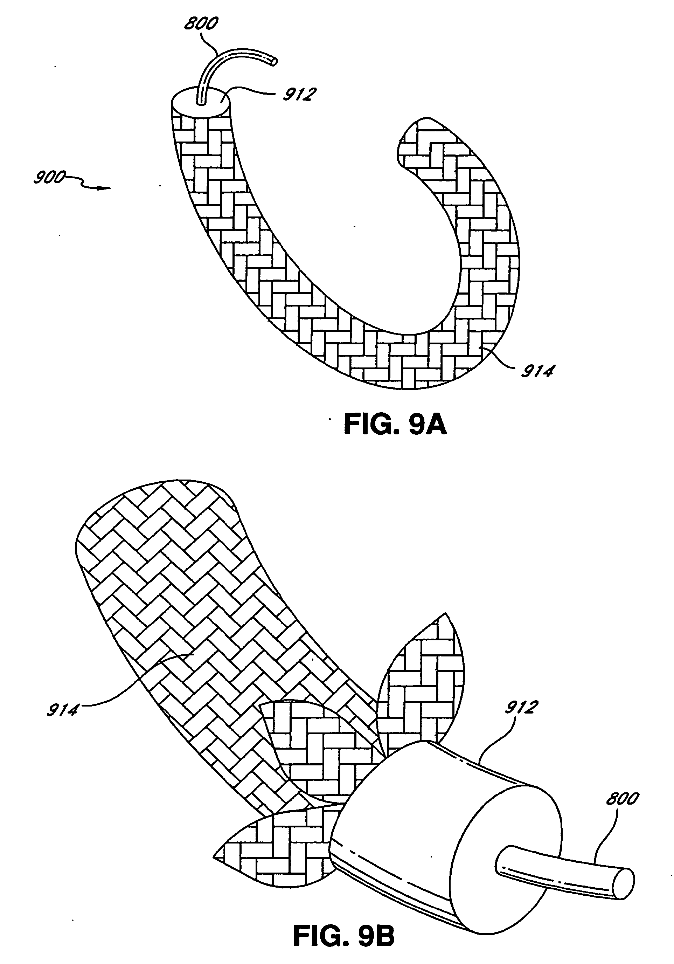

FIG. 9A is a perspective view illustrating portions of an annuloplasty ring 900 comprising the wire 800 shown in FIG. 8 not according to the invention. The wire 800 is covered by a flexible material 912 such as silicone rubber and a suturable material 914 such as woven polyester cloth, Dacron@, woven velour, polyurethane, polytetrafluoroethylene (PTFE), heparin-coated fabric, or other biocompatible material. In other examples, the suturable material 914 comprises a biological material such as bovine or equine pericardium, homograft, patient graft, or cell-seeded tissue. For illustrative purposes, portions of the flexible material 912 and the suturable material 914 are not shown in FIG. 9A to show the wire 800. However, in certain examples, the flexible material 912 and the suturable material 914 cover substantially the entire wire 800. Although not shown, in certain examples, the wire 800 is coated with an energy absorption enhancement material, as discussed above.

FIG. 9B is an enlarged perspective view of a portion of the annuloplasty ring 900 shown in FIG. 9A. For illustrative purposes, portions of the flexible material 912 are not shown to expose the wire 800 and portions of the suturable material 914 are shown peeled back to expose the flexible material 912. In certain examples, the diameter of the flexible material 912 is in a range between approximately 0.10 inches and approximately 0.15 inches. FIG. 9B shows the wire 800 substantially centered within the circumference of the flexible material 912. However, in certain examples, the wire 800 is offset within the circumference of the flexible material 912 to allow more space for sutures.

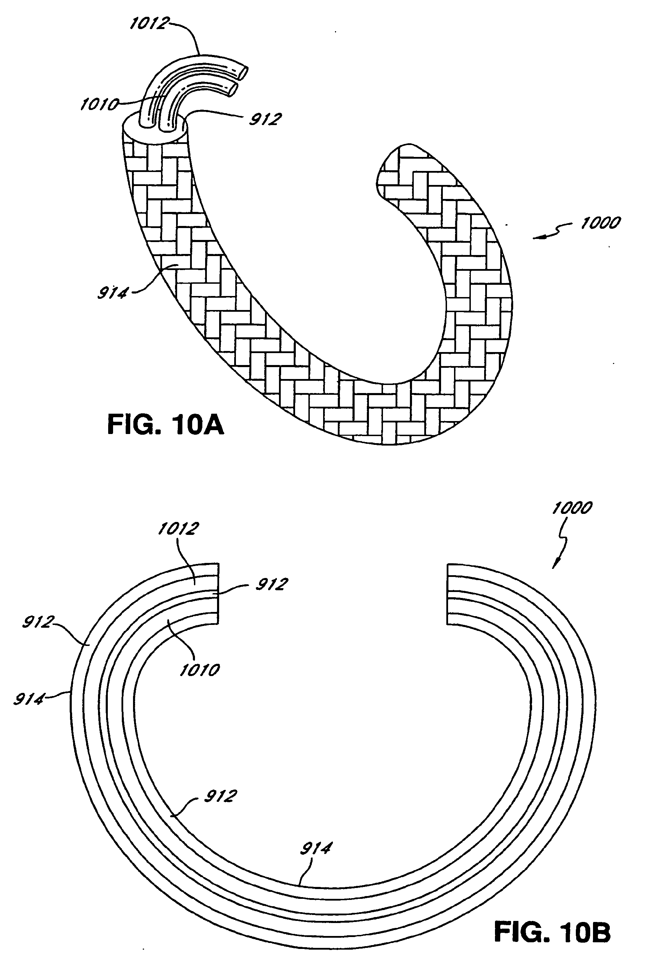

FIG. 10A is a perspective view illustrating portions of an annuloplasty ring 1000 configured to contract and expand not according to the invention. FIG. 10B is a top cross-sectional view of the annuloplasty ring 1000. As discussed above, after the annuloplasty ring 1000 has been contracted, it may become necessary to expand the annuloplasty ring 1000. For example, the annuloplasty ring 1000 may be implanted in a child with an enlarged heart. When the size of the heart begins to recover to its natural size, the annuloplasty ring 1000 can be contracted. Then, as the child gets older and the heart begins to grow, the annuloplasty ring 1000 can be enlarged as needed.

The annuloplasty ring 1000 comprises a first shape memory wire 1010 for contracting the annuloplasty ring 1000 and a second shape memory wire 1012 for expanding the annuloplasty ring 1000. The first and second shape memory wires, 1010, 1012 are covered by the flexible material 912 and the suturable material 914 shown in FIGS. 9A-9B. For illustrative purposes, portions of the flexible material 912 and the suturable material 914 are not shown in FIG. 10A to show the shape memory wires 1010, 1012. However, as schematically illustrated in FIG. 10B, in certain examples, the flexible material 912 and the suturable material 914 substantially cover the first and second shape memory wires 1010, 1012. As discussed below, the flexible material 912 operatively couples the first shape memory wire 1010 and the second shape memory wire 1012 such that a shape change in one will mechanically effect the shape of the other. The first and second shape memory wires 1010, 1012 each comprise a shape memory material, such as the shape memory materials discussed above. However, the first and second shape memory wires 1010, 1012 are activated at different temperatures.

In certain examples, the annuloplasty ring 1000 is heated to a first temperature that causes the first shape memory wire 1010 to transition to its austenite phase and contract to its memorized shape. At the first temperature, the second shape memory wire 1012 is in its martensite phase and is substantially flexible as compared the contracted first shape memory wire 1010. Thus, when the first shape memory wire 1010 transitions to its austenite phase, it exerts a sufficient force on the second shape memory wire 1012 through the flexible material 912 to deform the second shape memory wire 1012 and cause the annuloplasty ring 1000 to contract.

The annuloplasty ring 1000 can be expanded by heating the annuloplasty ring to a second temperature that causes the second shape memory wire 1012 to transition to its austenite phase and expand to its memorized shape. In certain embodiments, the second temperature is higher than the first temperature. Thus, at the second temperature, both the first and second shape memory wires 1010, 1012 are in their respective austenite phases. In certain such examples, the diameter of the second shape memory wire 1012 is sufficiently larger than the diameter of the first shape memory wire 1010 such that the second memory shape wire 1012 exerts a greater force to maintain its memorized shape in the austenite phase than the first shape memory wire 1010. Thus, the first shape memory wire 1010 is mechanically deformed by the force of the second memory shape wire 1012 and the annuloplasty ring 1000 expands.

In certain examples, the first memory shape wire 1010 is configured to contract by approximately 10% to 25% when transitioning to its austenite phase. In certain such examples, the first memory shape wire 1010 has an austenite start temperature Aₛ in a range between approximately 33 degrees Celsius and approximately 43 degrees Celsius, an austenite finish temperature A_{f} in a range between approximately 45 degrees Celsius and approximately 55 degrees Celsius, a martensite start temperature Mₛ less than approximately 30 degrees Celsius, and a martensite finish temperature M_{f} greater than approximately 20 degrees Celsius. In other examples, the austenite finish temperature A_{f} of the first memory shape wire 1010 is in a range between approximately 48.75 degrees Celsius and approximately 51.25 degrees Celsius.

In certain examples, the second memory shape wire 1012 is configured to expand by approximately 10% to 25% when transitioning to its austenite phase. In certain such examples, the second memory shape wire 1010 has an austenite start temperature Aₛ in a range between approximately 60 degrees Celsius and approximately 70 degrees Celsius, an austenite finish temperature A_{f} in a range between approximately 65 degrees Celsius and approximately 75 degrees Celsius, a martensite start temperature Mₛ less than approximately 30 degrees Celsius, and a martensite finish temperature M_{f} greater than approximately 20 degrees Celsius. In other examples, the austenite finish temperature A_{f} of the first memory shape wire 1010 is in a range between approximately 68.75 degrees Celsius and approximately 71.25 degrees Celsius.

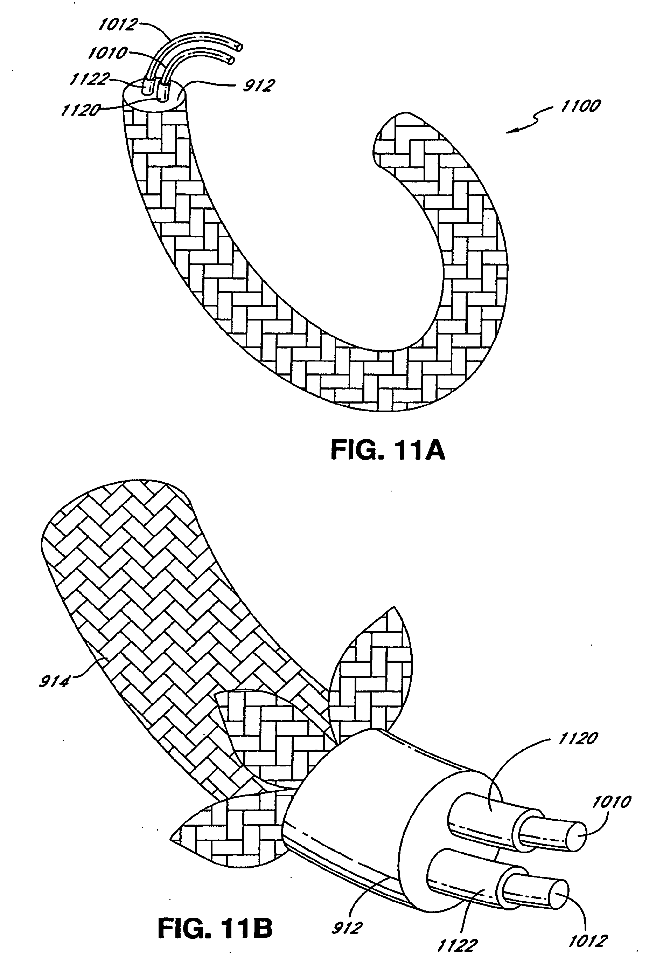

FIG. 11A is a perspective view illustrating portions of an annuloplasty ring 1100 comprising the first shape memory wire 1010 for contraction, the second shape memory wire 1012 for expansion, the flexible material 912 and the suturable material 914 shown in FIGS. 10A-10B. For illustrative purposes, portions of the flexible material 912 and the suturable material 914 are not shown in FIG. 11A to show the shape memory wires 1010, 1012. However, in certain examples, the flexible material 912 and the suturable material 914 substantially cover the first and second shape memory wires 1010, 1012. FIG. 11B is an enlarged perspective view of a portion of the annuloplasty ring 1100 shown in FIG. 11A. For illustrative purposes, portions of the flexible material 912 are not shown to expose the first and second shape memory wires 1010, 1012 and portions of the suturable material 914 are shown peeled back to expose the flexible material 912.

The first shape memory wire 1010 comprises a first coating 1120 and the second shape memory wire 1012 comprises a second coating 1122. In certain examples, the first coating 1120 and the second coating 1122 each comprise silicone tubing configured to provide suture attachment to a heart valve annulus. In certain other examples, the first coating 1120 and the second coating 1122 each comprise an energy absorption material, such as the energy absorption materials discussed above. In certain such examples, the first coating 1120 heats when exposed to a first form of energy and the second coating 1122 heats when exposed to a second form of energy. For example, the first coating 1120 may heat when exposed to MRI energy and the second coating 1122 may heat when exposed to HIFU energy. As another example, the first coating 1120 may heat when exposed to RF energy at a first frequency and the second coating 1122 may heat when exposed to RF energy at a second frequency. Thus, the first shape memory wire 1010 and the second shape memory wire 1012 can be activated independently such that one transitions to its austenite phase while the other remains in its martensite phase, resulting in contraction or expansion of the annuloplasty ring 1100.

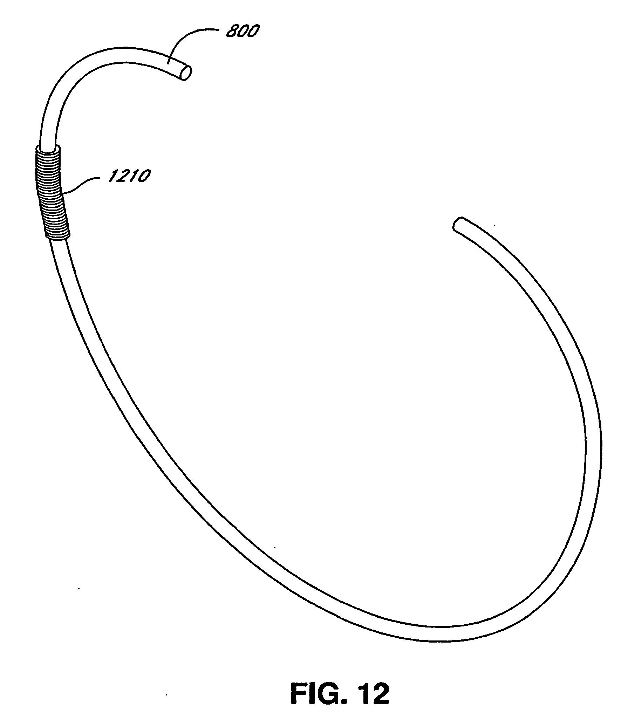

FIG. 12 is a perspective view of a shape memory wire 800, such as the wire 800 shown in FIG. 8, wrapped in an electrically conductive coil 1210 not according to the invention. The coil 1210 is wrapped around a portion of the wire 800 where it is desired to focus energy and heat the wire 800. In certain examples, the coil 1210 is wrapped around approximately 5% to approximately 15% of the wire 800. In other examples, the coil 1210 is wrapped around approximately 15% to approximately 70% of the wire 800. In other embodiments, the coil 1210 is wrapped around substantially the entire wire 800. Although not shown, in certain examples, the wire 800 also comprises a coating comprising an energy absorption material, such as the energy absorption materials discussed above. The coating may or may not be covered by the coil 1210.

As discussed above, an electrical current can be non-invasively induced in the coil 1210 using electromagnetic energy. For example, in certain examples, a handheld or portable device (not shown) comprising an electrically conductive coil generates an electromagnetic field that non-invasively penetrates the patient's body and induces a current in the coil 1210. The electrical current causes the coil 1210 to heat. The coil 1210, the wire 800 and the coating (if any) are thermally conductive so as to transfer the heat or thermal energy from the coil 1210 to the wire 800. Thus, thermal energy can be directed to the wire 800, or portions thereof, while reducing thermal damage to surrounding tissue.

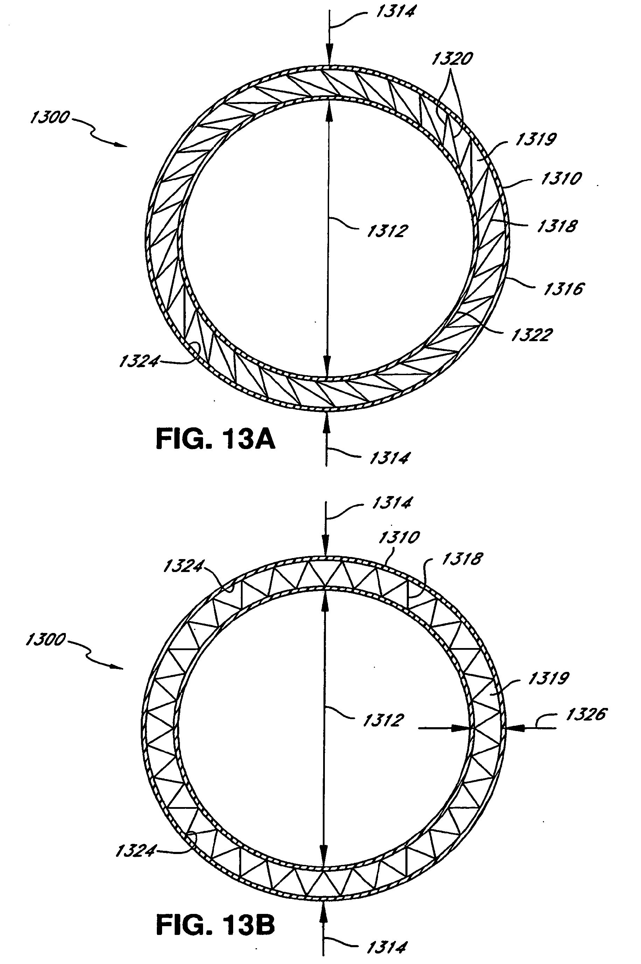

FIGS. 13A and 13B show an annuloplasty ring 1310 having a nominal inner diameter or transverse dimension indicated by arrow 1312 and a nominal outer diameter or transverse dimension indicated by arrows 1314. The ring 1310 includes a tubular member 1316 having a substantially round transverse cross section with an internal shape memory member 1318 disposed within an inner chamber 1319 of the tubular member 1316. The internal shape memory member 1318 is a ribbon or wire bent into a series of interconnected segments 1320. Upon heating of the tubular member 1316 and the internal shape memory member 1318, the inner transverse dimension 1312 becomes smaller due to axial shortening of the tubular member 1316 and an inward radial force applied to an inner chamber surface 1322 of the tubular member 1316 by the internal shape memory member 1318. The internal shape memory member 1318 is expanded upon heating such that the ends of segments 1320 push against the inner chamber surface 1322 and outer chamber surface 1324, as shown by arrow 1326 in FIG. 13B, and facilitate radial contraction of the inner transverse dimension 1312. Thus, activation of the internal shape memory member 1318 changes the relative distance between the against the inner chamber surface 1322 and outer chamber surface 1324.

Although not shown in FIGS. 13A or 13B, The inner shape memory member 1318 may also have a heating energy absorption enhancement material, such as one or more of the energy absorption enhancement materials discussed above, disposed about it within the inner chamber 1319. The energy absorption material may also be coated on an outer surface and/or an inner surface of the tubular member 1316. The inner transverse dimension 1312 of the ring 1310 in FIG. 13B is less than the inner transverse dimension 1312 of the ring 1310 shown in FIG. 13A. However, according to certain examples, the outer transverse dimension 1314 is substantially constant in both FIGS. 13A and 13B.

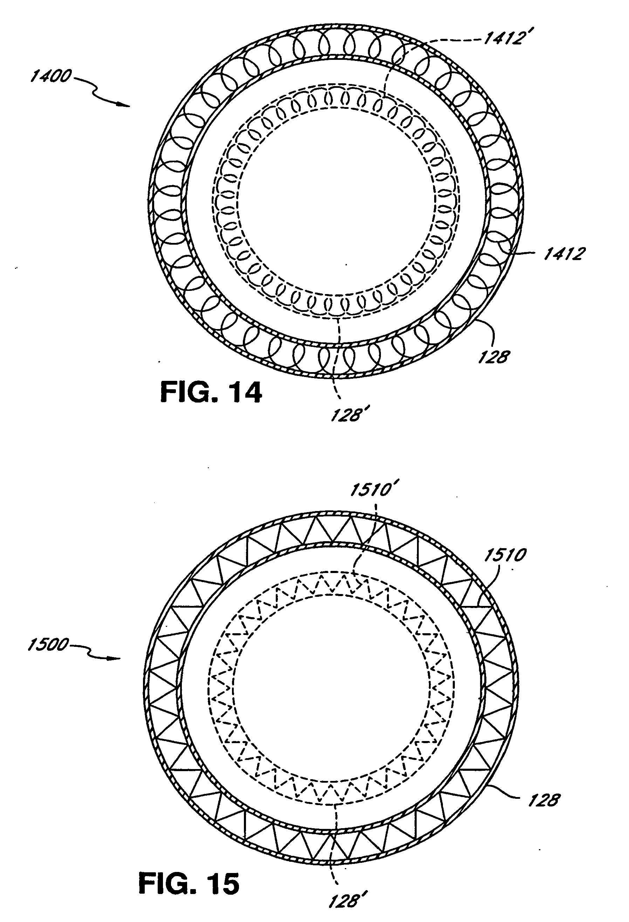

For some indications, it may be desirable for an adjustable annuloplasty ring to have some compliance in order to allow for expansion and contraction of the ring in concert with the expansion and contraction of the heart during the beating cycle or with the hydrodynamics of the pulsatile flow through the valve during the cycle. As such, it may be desirable for an entire annuloplasty ring, or a section or sections thereof, to have some axial flexibility to allow for some limited and controlled expansion and contraction under clinical conditions. FIGS. 14 and 15 illustrate examples of adjustable annuloplasty rings that allow some expansion and contraction in a deployed state.

FIG. 14 shows an annuloplasty ring 1400 that is constructed in such a way that it allows mechanical expansion and compression of the ring 1400 under clinical conditions. The ring 1400 includes a coil 1412 made of a shape memory material, such as one or more of the shape memory materials discussed above. The shape memory material or other portion of the ring 1400 may be coated with an energy absorption material, such as the energy absorption materials discussed above. The coil 1412 may have a typical helical structure of a normal spring wire coil, or alternatively, may have another structure such as a ribbon coil. In certain embodiments, the coil 1412 is surrounded by a suturable material 128, such as Dacron@ or the other suturable materials discussed herein. The coiled structure or configuration of the coil 1412 allows the ring 1400 to expand and contract slightly when under physiological pressures and forces from heart dynamics or hydrodynamics of blood flow through a host heart valve.

For examples where the coil 1412 is made of NiTi alloy or other shape memory material, the ring 1400 is responsive to temperature changes which may be induced by the application of heating energy on the coil 1412. In certain examples, if the temperature is raised, the coil 1412 will contract axially or circumferentially such that an inner transverse dimension of the ring 1400 decreases, as shown by the dashed lines in FIG. 14. In FIG. 14, reference 1412' represents the coil 1412 in its contracted state and reference 128' represents the suturable material 128 in its contracted state around the contracted coil 1412'. In addition, or in other embodiments, the coil 1412 expands axially or circumferentially such that the inner transverse dimension of the ring 1400 increases. Thus, in certain examples, the ring 1400 can be expanded and contracted by applying invasive or non-invasive energy thereto.

FIG. 15 illustrates another examples of an adjustable annuloplasty ring 1500 that has dynamic compliance with dimensions, features and materials that may be the same as or similar to those of ring 1400. However, the ring 1500 has a zig-zag ribbon member 1510 in place of the coil 1412 in the embodiment of FIG. 14. In certain examples, if the temperature is raised, the ribbon member 1510 will contract axially or circumferentially such that an inner transverse dimension of the ring 1500 decreases, as shown by the dashed lines in FIG. 15. In FIG. 15, reference 1510' represents the ribbon member 1510 in its contracted state and reference 128' represents the suturable material 128 in its contracted state around the contracted ribbon member 1510'. In addition, or in other examples, the ribbon member 1510 expands axially or circumferentially such that the inner transverse dimension of the ring 1500 increases. Thus, in certain examples, the ring 1500 can be expanded and contracted by applying invasive or non-invasive energy thereto.

The examples of FIGS. 14 and 15 may have a substantially circular configuration as shown in the figures, or may have D-shaped or C-shaped configurations as shown with regard to other examples discussed above. In certain examples, the features, dimensions and materials of rings 1400 and 1500 are the same as or similar to the features, dimensions and materials of the annuloplasty ring 400 discussed above.

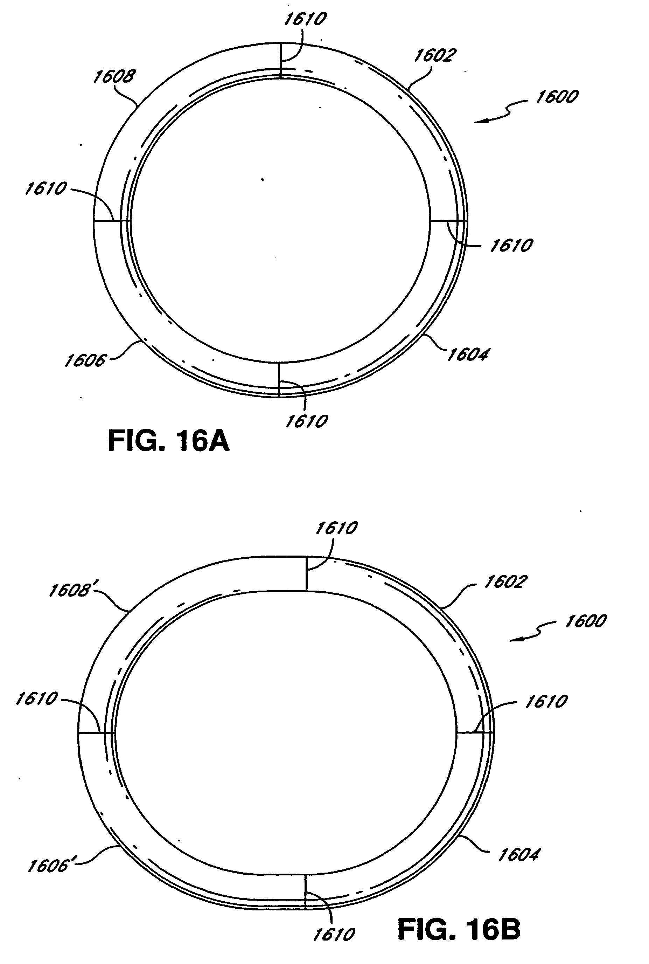

FIGS. 16A and 16B illustrate an annuloplasty ring 1600 according to certain examples that has a substantially circular shape or configuration when in the non-activated state shown in FIG. 16A. The ring 1600 comprises shape memory material or materials which are separated into a first temperature response zone 1602, a second temperature response zone 1604, a third temperature response zone 1606 and a fourth temperature response zone 1608. The zones are axially separated by boundaries 1610. Although the ring 1600 is shown with four zones 1602, 1604, 1606, 1608, an artisan will recognize from the disclosure herein that other embodiments may include two or more zones of the same or differing lengths. For example, one examples of an annuloplasty ring 1600 includes approximately three to approximately eight temperature response zones.

In certain examples, the shape memory materials of the various temperature response zones 1602, 1604, 1606, 1608 are selected to have temperature responses and reaction characteristics such that a desired shape and configuration can be achieved in vivo by the application of invasive or non-invasive energy, as discussed above. In addition to general contraction and expansion changes, more subtle changes in shape and configuration for improvement or optimization of valve function or hemodynamics may be achieved with such examples.

According to certain examples, the first zone 1602 and second zone 1604 of the ring 1600 are made from a shape memory material having a first shape memory temperature response. The third zone 1606 and fourth zone 1608 are made from a shape memory material having a second shape memory temperature response. In certain examples, the four zones comprise the same shape memory material, such as NiTi alloy or other shape memory material as discussed above, processed to produce the varied temperature response in the respective zones. In other examples, two or more of the zones may comprise different shape memory materials. Certain examples include a combination of shape memory alloys and shape memory polymers in order to achieve the desired results.

According to certain examples, FIG. 16B shows the ring 1600 after heat activation such that it comprises expanded zones 1606', 1608' corresponding to the zones 1606, 1608 shown in FIG. 16A. As schematically shown in FIG. 16A, activation has expanded the zones 1606', 1608' so as to increase the axial lengths of the segments of the ring 1600 corresponding to those zones. In addition, or in other examples, the zones 1606 and 1608 are configured to contract by a similar percentage instead of expand. In other examples, the zones 1602, 1604, 1606, 1608 are configured to each have a different shape memory temperature response such that each segment corresponding to each zone 1602, 1604, 1606, 1608 could be activated sequentially.

FIG. 16B schematically illustrates that the zones 1606', 1608' have expanded axially (i.e., from their initial configuration as shown by the zones 1606, 1608 in FIG. 16A). In certain examples, the zones 1602, 1604 are configured to be thermally activated to remember a shape memory dimension or size upon reaching a temperature in a range between approximately 51 degrees Celsius and approximately 60 degrees Celsius. In certain such examples, the zones 1606 and 1608 are configured to respond at temperatures in a range between approximately 41 degrees Celsius and approximately 48 degrees Celsius. Thus, for example, by applying invasive or non-invasive energy, as discussed above, to the ring 1600 until the ring 1600 reaches a temperature of approximately 41 degrees Celsius to approximately 48 degrees Celsius, the zones 1606, 1608 will respond by expanding or contracting by virtue of the shape memory mechanism, and the zones 1602, 1604 will not.

In certain other examples, the zones 1602, 1604 are configured to expand or contract by virtue of the shape memory mechanism at a temperature in a range between approximately 50 degrees Celsius and approximately 60 degrees Celsius. In certain such examples, the zones 1606, 1608 are configured to respond at a temperature in a range between approximately 39 degrees Celsius and approximately 45 degrees Celsius.

In certain examples, the materials, dimensions and features of the annuloplasty ring 1600 and the corresponding zones 1602, 1604, 1606, 1608 have the same or similar features, dimensions or materials as those of the other ring embodiments discussed above. In certain examples, the features of the annuloplasty ring 1600 are added to the embodiments discussed above.

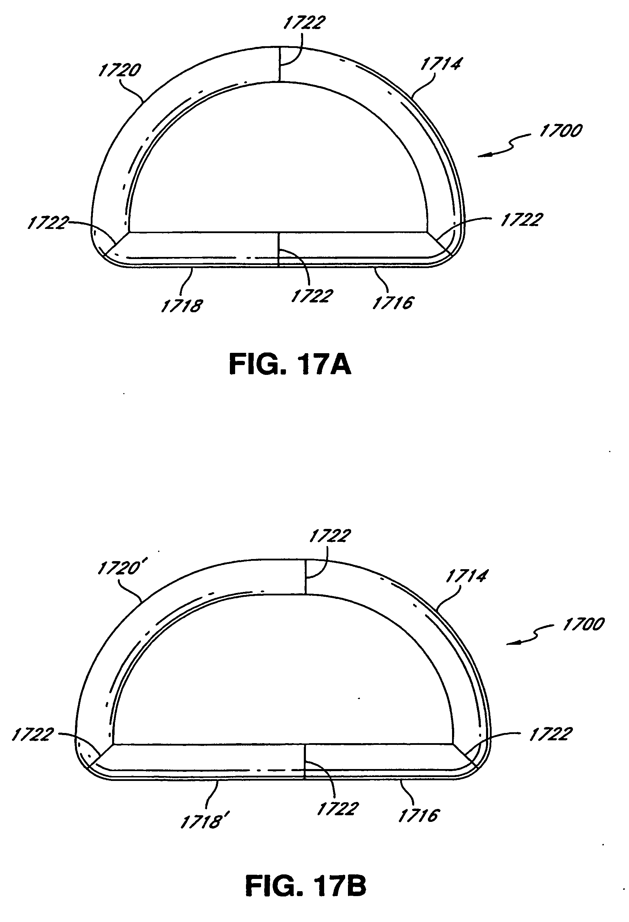

FIGS. 17A and 17B illustrate an annuloplasty ring 1700 not according to the invention that is similar to the annuloplasty ring 1600 discussed above, but having a "D-shaped" configuration. The ring 1700 comprises shape memory material or materials which are separated into a first temperature response zone 1714, a second temperature response zone 1716, a third temperature response zone 1718 and a fourth temperature response zone 1720. The segments defined by the zones 1714, 1716, 1718, 1720 are separated by boundaries 1722. Other than the D-shaped configuration, the ring 1700 according to certain embodiments has the same or similar features, dimensions and materials as the features, dimension and materials of the ring 1600 discussed above.

According to certain examples, FIG. 17B shows the ring 1700 after heat activation such that it comprises expanded zones 1718', 1720' corresponding to the zones 1718, 1720 shown in FIG. 17A. As schematically shown in FIG. 17B, activation has expanded the zones 1718', 1720' by virtue of the shape memory mechanism. The zones 1718, 1720 could also be selectively shrunk or contracted axially by virtue of the same shape memory mechanism for an example having a remembered shape smaller than the nominal shape shown in FIG. 17A. The transverse cross sections of the rings 1600 and 1700 are substantially round, but can also have any other suitable transverse cross sectional configuration, such as oval, square, rectangular or the like.

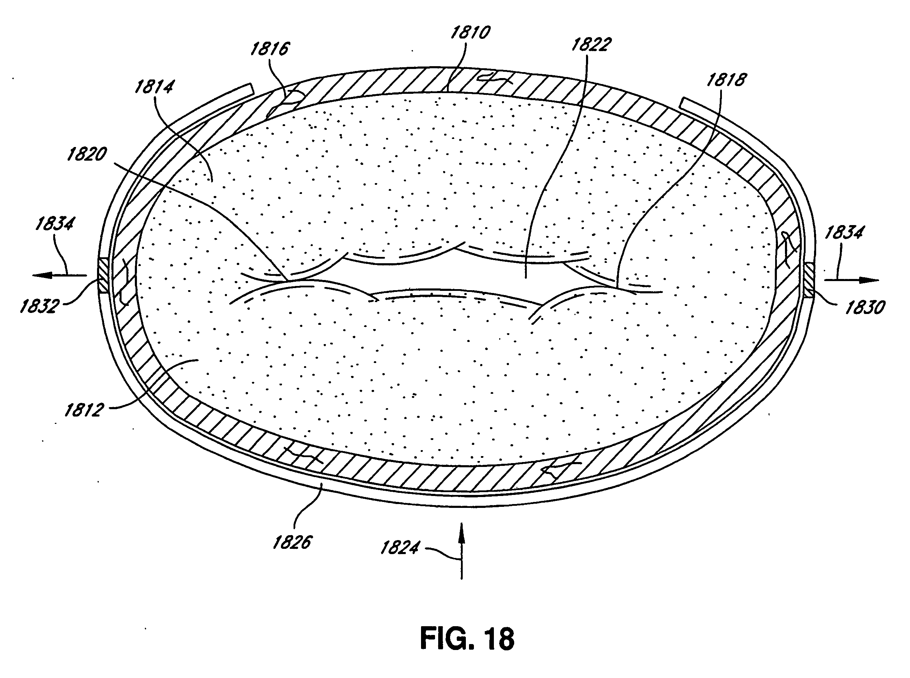

In certain situations, it is advantageous to reshape a heart valve annulus in one dimension while leaving another dimension substantially unchanged or reshaped in a different direction. For example, FIG. 18 is a sectional view of a mitral valve 1810 having an anterior (aortic) leaflet 1812, a posterior leaflet 1814 and an annulus 1816. The anterior leaflet 1812 and the posterior leaflet 1814 meet at a first commissure 1818 and a second commissure 1820. When healthy, the annulus 1816 encircles the leaflets 1812, 1814 and maintains their spacing to provide closure of a gap 1822 during left ventricular contraction. When the heart is not healthy, the leaflets 1812, 1814 do not achieve sufficient coaptation to close the gap 1822, resulting in regurgitation. In certain examples, the annulus 1816 is reinforced so as to push the anterior leaflet 1812 and the posterior leaflet 1814 closer together without substantially pushing the first commissure 1818 and the second commissure 1820 toward one another.

FIG. 18 schematically illustrates an exemplary annuloplasty ring 1826 comprising shape memory material configured to reinforce the annulus 1816 not according to the invention. For illustrative purposes, the annuloplasty ring 1826 is shown in an activated state wherein it has transformed to a memorized configuration upon application of invasive or non-invasive energy, as described herein. While the annuloplasty ring 1826 is substantially C-shaped, an artisan will recognize from the disclosure herein that other shapes are possible including, for example, a continuous circular, oval or D-shaped ring.

In certain examples, the annuloplasty ring 1826 comprises a first marker 1830 and a second marker 1832 that are aligned with the first commissure 1818 and the second commissure 1820, respectively, when the annuloplasty ring 1826 is implanted around the mitral valve 1810. In certain examples, the first marker 1830 and the second marker 1832 comprise materials that can be imaged in-vivo using standard imaging techniques. For example, in certain examples, the markers 1830 comprise radiopaque markers or other imaging materials, as is known in the art. Thus, the markers 1830, 1832 can be used for subsequent procedures for alignment with the annuloplasty ring 1826 and/or the commissures 1818, 1820. For example, the markers 1830, 1832 can be used to align a percutaneous energy source, such as a heated balloon inserted through a catheter, with the annuloplasty ring 1826.

When the shape memory material is activated, the annuloplasty ring 1826 contracts in the direction of the arrow 1824 to push the anterior leaflet 1812 toward the posterior leaflet 1814. Such anterior/posterior contraction improves the coaptation of the leaflets 1812, 1814 such that the gap 1824 between the leaflets 1812, 1814 sufficiently closes during left ventricular contraction. In certain embodiments, the annuloplasty ring 1826 also expands in the direction of arrows 1834. Thus, the first commissure 1818 and the second commissure 1820 are pulled away from each other, which draws the leaflets 1812, 1814 closer together and further improves their coaptation. However, in certain other examples, the annuloplasty ring does not expand in the direction of the arrows 1834. In certain such embodiments, the distance between the lateral portions of the annuloplasty ring 1826 between the anterior portion and the posterior portion (e.g., the lateral portions approximately correspond to the locations of the markers 1830, 1832 in the embodiment shown in FIG. 18) remains substantially the same after the shape memory material is activated.

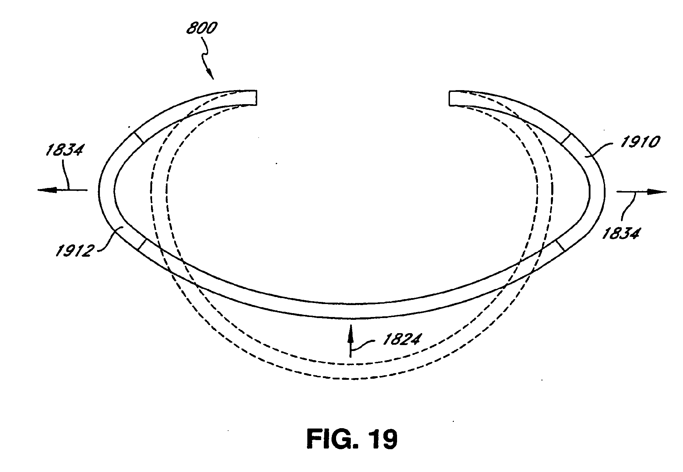

FIG. 19 is a schematic diagram of a substantially C-shaped wire comprising a shape memory material configured to contract in a first direction and expand in a second direction not according to the invention. Suitable shape memory materials include shape memory polymers or shape memory alloys including, for example, ferromagnetic shape memory alloys, as discussed above. FIG. 19 schematically illustrates the wire 800 in its activated configuration or memorized shape. For illustrative purposes, the wire 800 is shown relative to dashed lines representing its deformed shape or configuration when implanted into a body before activation.

When the shape memory material is activated, the wire 800 is configured to respond by contracting in a first direction as indicated by arrow 1824. In certain embodiments, the wire 800 also expands in a second direction as indicated by arrows 1834. Thus, the wire 800 is usable by the annuloplasty ring 1826 shown in FIG. 18 to improve the coaptation of the leaflets 1812, 1814 by contracting the annulus 1816 in the anterior/posterior direction. In certain examples, the anterior/posterior contraction is in a range between approximately 10% and approximately 20%. In certain examples, only a first portion 1910 and a second portion 1912 of the wire 800 comprise the shape memory material. When the shape memory material is activated, the first portion 1910 and the second portion 1912 of the wire 800 are configured to respond by transforming to their memorized configurations and reshaping the wire 800 as shown.

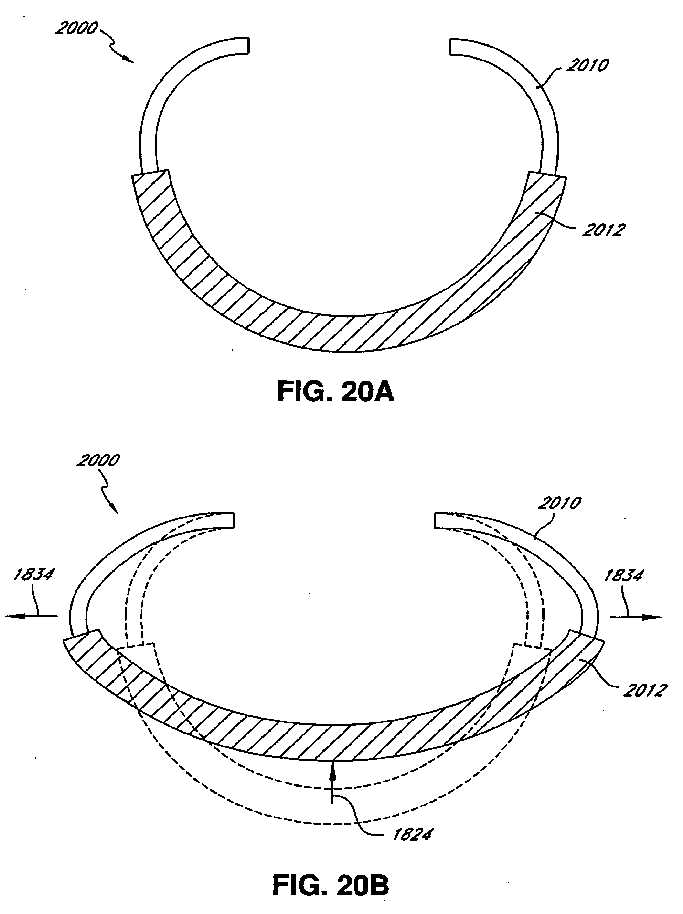

FIGS. 20A and 20B are schematic diagrams of a body member 2000 according to certain examples usable by an annuloplasty ring, such as the annuloplasty ring 1826 shown in FIG. 18. Although not shown, in certain examples, the body member 2000 is covered by a flexible material such as silicone rubber and a suturable material such as woven polyester cloth, Dacron@, woven velour, polyurethane, polytetrafluoroethylene (PTFE), heparin-coated fabric, or other biocompatible material, as discussed above.

The body member 2000 comprises a wire 2010 and a shape memory tube 2012. As used herein, the terms "tube," "tubular member" and "tubular structure" are broad terms having at least their ordinary and customary meaning and include, for example, hollow elongated structures that may in cross-section be cylindrical, elliptical, polygonal, or any other shape. Further, the hollow portion of the elongated structure may be filled with one or more materials that may be the same as and/or different than the material of the elongated structure. In certain examples, the wire 2010 comprises a metal or metal alloy such as stainless steel, titanium, platinum, combinations of the foregoing, or the like. As used herein, the term "wire" is a broad term having at least its ordinary and customary meaning and includes, for example, solid, hollow or tubular elongated structures that may in cross-section be cylindrical, elliptical, polygonal, or any other shape, including a substantially flat ribbon shape. In certain examples, the shape memory tube 2012 comprises shape memory materials formed in a tubular structure through which the wire 2010 is inserted. In certain other examples, the shape memory tube 2012 comprises a shape memory material coated over the wire 2010. Suitable shape memory materials include shape memory polymers or shape memory alloys including, for example, ferromagnetic shape memory alloys, as discussed above. Although not shown, in certain examples, the body member 2000 comprises an energy absorption enhancement material, as discussed above.

FIG. 20A schematically illustrates the body member 2000 in a first configuration or shape and FIG. 20B schematically illustrates the body member 2000 in a second configuration or shape after the shape memory tube has been activated. For illustrative purposes, dashed lines in FIG. 20B also show the first configuration of the body member 2000. When the shape memory material is activated, the shape memory tube 2012 is configured to respond by contracting in a first direction as indicated by arrow 1824. In certain examples, the shape memory tube 2012 is also configured to expand in a second direction as indicated by arrows 1834. The transformation of the shape memory tube 2012 exerts a force on the wire 2010 to change its shape. Thus, the body member 2000 is usable by the annuloplasty ring 1826 shown in FIG. 18 to pull the commissures 1818, 1820 further apart and push the leaflets 1812, 1814 closer together to improve coaptation.

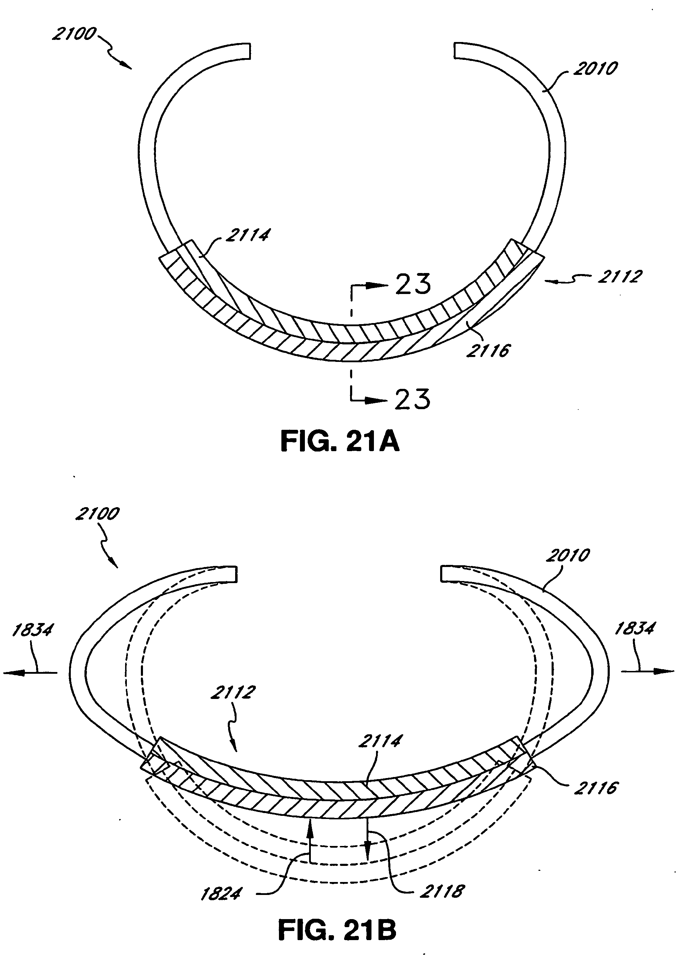

FIGS. 21A and 21B are schematic diagrams of a body member 2100 usable by an annuloplasty ring, such as the annuloplasty ring 1826 shown in FIG. 18. Although not shown, in certain examples, the body member 2100 is covered by a flexible material such as silicone rubber and a suturable material such as woven polyester cloth, Dacron@, woven velour, polyurethane, polytetrafluoroethylene (PTFE), heparin-coated fabric, or other biocompatible material, as discussed above.

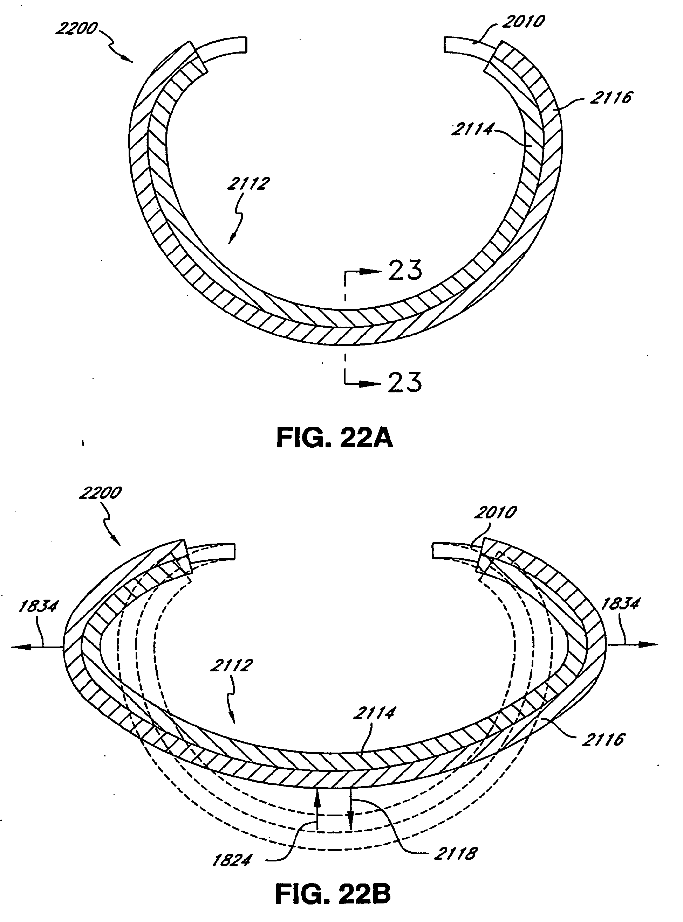

The body member 2100 comprises a wire 2010, such as the wire 2010 shown in FIGS. 20A and 20B and a shape memory tube 2112. As schematically illustrated in FIGS. 21A and 21B, the shape memory tube 2112 is sized and configured to cover a certain percentage of the wire 2010. However, an artisan will recognize from the disclosure herein that in other examples the shape memory tube 2112 may cover other percentages of the wire 2010. Indeed, FIGS. 22A and 22B schematically illustrate another examples of a body member 2200 comprising a shape memory tube 2112 covering a substantial portion of a wire 2010. The amount of coverage depends on such factors as the particular application, the desired shape change, the shape memory materials used, the amount of force to be exerted by the shape memory tube 2112 when changing shape, combinations of the foregoing, and the like. For example, in certain examples where, as in FIGS. 22A and 22B, the shape memory tube 2112 covers a substantial portion of a wire 2010, portions of the shape memory tube 2112 are selectively heated to reshape the wire 2010 at a particular location. In certain such examples, HIFU energy is directed towards, for example, the left side of the shape memory tube 2112, the right side of the shape memory tube 2112, the bottom side of the shape memory tube 2112, or a combination of the foregoing to activate only a portion of the shape memory tube 2112. Thus, the body member 2200 can be reshaped one or more portions at a time to allow selective adjustments.

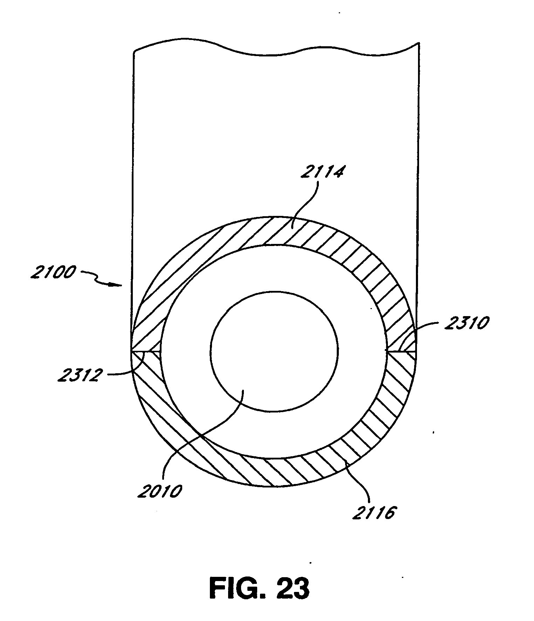

In certain examples, the shape memory tube 2112 comprises a first shape memory material 2114 and a second shape memory material 2116 formed in a tubular structure through which the wire 2010 is inserted. In certain such examples, the first shape memory material 2114 and the second shape memory material 2116 are each configured as a semi-circular portion of the tubular structure. For example, FIG. 23 is a transverse cross-sectional view of the body member 2100. As schematically illustrated in FIG. 23, the first shape memory material 2114 and the second shape memory material 2116 are joined at a first boundary 2310 and a second boundary 2312. In certain examples, silicone tubing (not shown) holds the first shape memory material 2114 and the second shape memory material 2116 together. In certain other examples, the first shape memory material 2114 and the second shape memory material 2116 each comprise a shape memory coating covering opposite sides of the wire 2010. Suitable shape memory materials include shape memory polymers or shape memory alloys including, for example, ferromagnetic shape memory alloys, as discussed above. Although not shown, in certain examples the body member 2100 comprises an energy absorption enhancement material, as discussed above.

FIG. 21A schematically illustrates the body member 2100 in a first configuration or shape before the first shape memory material 2114 and the second shape memory material 2116 are activated. In certain examples, the first shape memory material 2114 and the second shape memory material 2116 are configured to be activated or return to their respective memorized shapes at different temperatures. Thus, the first shape memory material 2114 and the second shape memory material 2116 can be activated at different times to selectively expand and/or contract the body member 2100. For example, in certain examples, the second shape memory material 2116 is configured to be activated at a lower temperature than the first shape memory material 2114.

FIG. 21B schematically illustrates the body member 2100 in a second configuration or shape after the second shape memory material 2116 has been activated. For illustrative purposes, dashed lines in FIG. 21B also show the first configuration of the body member 2100. When the second shape memory material 2116 is activated, it responds by bending the body member 2100 in a first direction as indicated by arrow 1824. In certain examples, activation also expands the body member 2100 in a second direction as indicated by arrows 1834. Thus, the body member 2100 is usable by the annuloplasty ring 1826 shown in FIG. 18 to pull the commissures 1818, 1820 further apart and push the leaflets 1812, 1814 closer together to improve coaptation.

In certain examples, the first shape memory material 2114 can then be activated to bend the body member 2100 opposite to the first direction as indicated by arrow 2118. In certain such examples, the body member 2100 is reshaped to the first configuration as shown in FIG. 21A (or the dashed lines in FIG. 21 B). Thus, for example, if the size of the patient's heart begins to grow again (e.g., due to age or illness), the body member 2100 can be enlarged to accommodate the growth. In certain other examples, activation of the first shape memory material 2114 further contracts the body member 2100 in the direction of the arrow 1824. In certain embodiments, the first shape memory material 2114 has an austenite start temperature Aₛ in a range between approximately 42 degrees Celsius and approximately 50 degrees Celsius and the second shape memory material 2116 has an austenite start temperature Aₛ in a range between approximately 38 degrees Celsius and 41 degrees Celsius.

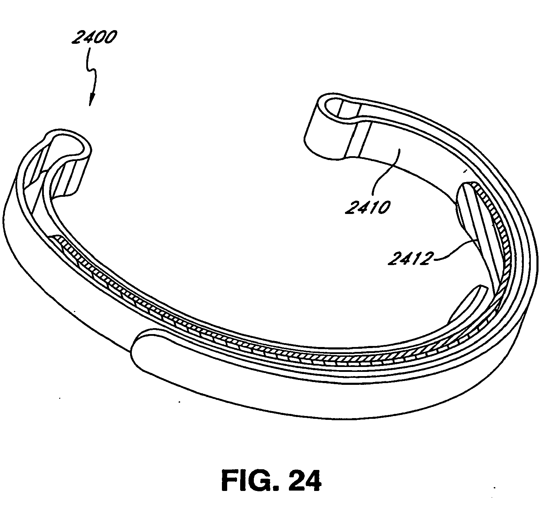

FIG. 24 is a perspective view of a body member 2400 usable by an annuloplasty ring comprising a first shape memory band 2410 and a second shape memory band 2412. Suitable shape memory materials for the bands 2410, 2412 include shape memory polymers or shape memory alloys including, for example, ferromagnetic shape memory alloys, as discussed above. Although not shown, in certain examples, the body member 2100 comprises an energy absorption enhancement material, as discussed above. Although not shown, in certain examples, the body member 2100 is covered by a flexible material such as silicone rubber and a suturable material such as woven polyester cloth, Dacron®, woven velour, polyurethane, polytetrafluoroethylene (PTFE), heparin-coated fabric, or other biocompatible material, as discussed above.

The first shape memory band 2410 is configured to loop back on itself to form a substantially C-shaped configuration. However, an artisan will recognize from the disclosure herein that the first shape memory band 2410 can be configured to loop back on itself in other configurations including, for example, circular, D-shaped, or other curvilinear configurations. When activated, the first shape memory band 2410 expands or contracts such that overlapping portions of the band 2410 slide with respect to one another, changing the overall shape of the body member 2400. The second shape memory band 2412 is disposed along a surface of the first shape memory band 2410 such that the second shape memory band 2412 is physically deformed when the first shape memory band 2410 is activated, and the first shape memory band 2410 is physically deformed when the second shape memory band 2412 is activated.

As shown in FIG. 24, in certain examples at least a portion of the second shape memory band 2412 is disposed between overlapping portions of the first shape memory band 2410. An artisan will recognize from the disclosure herein, however, that the second shape memory band 2412 may be disposed adjacent to an outer surface or an inner surface of the first shape memory band 2410 rather than between overlapping portions of the first shape memory band 2410. When the second shape memory band 2412 is activated, it expands or contracts so as to slide with respect to the first shape memory band 2410. In certain embodiments, the first shape memory band 2410 and the second shape memory band 2412 are held in relative position to one another by the flexible material and/or suturable material discussed above.

While the first shape memory band 2410 and the second shape memory band 2412 shown in FIG. 24 are substantially flat, an artisan will recognize from the disclosure herein that other shapes are possible including, for example, rod-shaped wire. However, in certain examples the first shape memory band 2410 and the second shape memory band 2412 advantageously comprise substantially flat surfaces configured to guide one another during expansion and/or contraction. Thus, the surface area of overlapping portions of the first shape memory band 2410 and/or the second shape memory band 2412 guide the movement of the body member 2400 in a single plane and reduce misalignment (e.g., twisting or moving in a vertical plane) during shape changes. The surface area of overlapping portions also advantageously increases support to a heart valve by reducing misalignment during beating of the heart.

An artisan will recognize from the disclosure herein that certain embodiments of the body member 2400 may not comprise either the first shape memory band 2410 or the second shape memory band 2412. For example, in certain examples the body member 2400 does not include the second shape memory band 2412 and is configured to expand and/or contract by only activating the first shape memory band 2410. Further, an artisan will recognize from the disclosure herein that either the first band 2410 or the second band 2412 may not comprise a shape memory material. For example, the first band 2410 may titanium, platinum, stainless steel, combinations of the foregoing, or the like and may be used with or without the second band 2412 to support a coronary valve annulus.

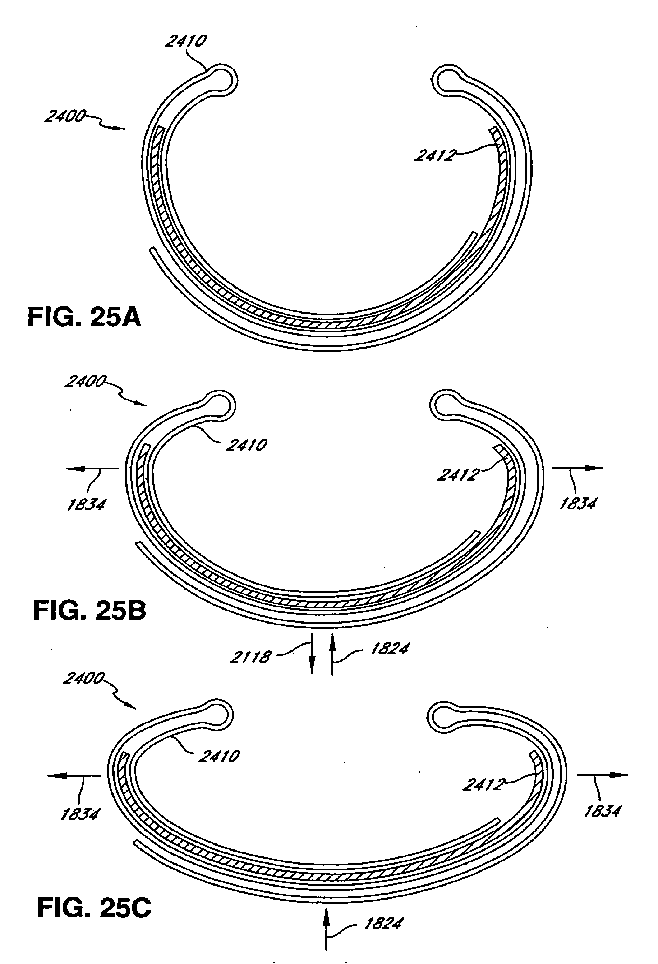

As schematically illustrated in FIGS. 25A-25C, in certain examples the body member 2400 is configured to change shape at least twice by activating both the first shape memory band 2410 and the second shape memory band 2412. FIG. 25A schematically illustrates the body member 2400 in a first configuration or shape before the first shape memory band 2410 or the second shape memory band 2412 are activated. In certain examples, the first shape memory band 2410 and the second shape memory band 2412 are configured to be activated or return to their respective memorized shapes at different temperatures. Thus, the first shape memory band 2410 and the second shape memory band 2412 can be activated at different times to selectively expand and/or contract the body member 2400. For example (and for purposes of discussing FIGS. 25A-25C), in certain examples, the first shape memory band 2410 is configured to be activated at a lower temperature than the second shape memory band 2412. However, an artisan will recognize from the disclosure herein that in other examples the second shape memory band 2412 may be configured to be activated at a lower temperature than the first shape memory band 2410.

FIG. 25B schematically illustrates the body member 2400 in a second configuration or shape after the first shape memory band 2410 has been activated. When the first shape memory band 2410 is activated, it responds by bending the body member 2400 in a first direction as indicated by arrow 1824. In certain embodiments, the activation also expands the body member 2400 in a second direction as indicated by arrows 1834. Thus, the body member 2400 is usable by the annuloplasty ring 1826 shown in FIG. 18 to pull the commissures 1818, 1820 further apart and push the leaflets 1812, 1814 closer together to improve coaptation.

In certain examples, the second shape memory band 2412 can then be activated to further contract the body member 2400 in the direction of the arrow 1824 and, in certain examples, further expand the body member 2400 in the direction of arrows 1834. In certain such examples, activating the second shape memory band 2412 reshapes the body member 2400 to a third configuration as shown in FIG. 25C. Thus, for example, as the patient's heart progressively heals and reduces in size, the body member 2400 can be re-sized to provide continued support and improved leaflet coaptation. In certain other examples, activation of the second shape memory band 2412 bends the body member 2400 opposite to the first direction as indicated by arrow 2118. In certain such examples, activating the second shape memory band 2412 reshapes the body member 2400 to the first configuration as shown in FIG. 25A. Thus, for example, if the size of the patient's heart begins to grow again (e.g., due to age or illness), the body member 2400 can be re-sized to accommodate the growth.

In certain annuloplasty ring examples, flexible materials and/or suturable materials used to cover shape memory materials also thermally insulate the shape memory materials so as to increase the time required to activate the shape memory materials through application of thermal energy. Thus, surrounding tissue is exposed to the thermal energy for longer periods of time, which may result in damage to the surrounding tissue. Therefore, in certain examples of the invention, thermally conductive materials are configured to penetrate the flexible materials and/or suturable materials so as to deliver thermal energy to the shape memory materials such that the time required to activate the shape memory materials is decreased.

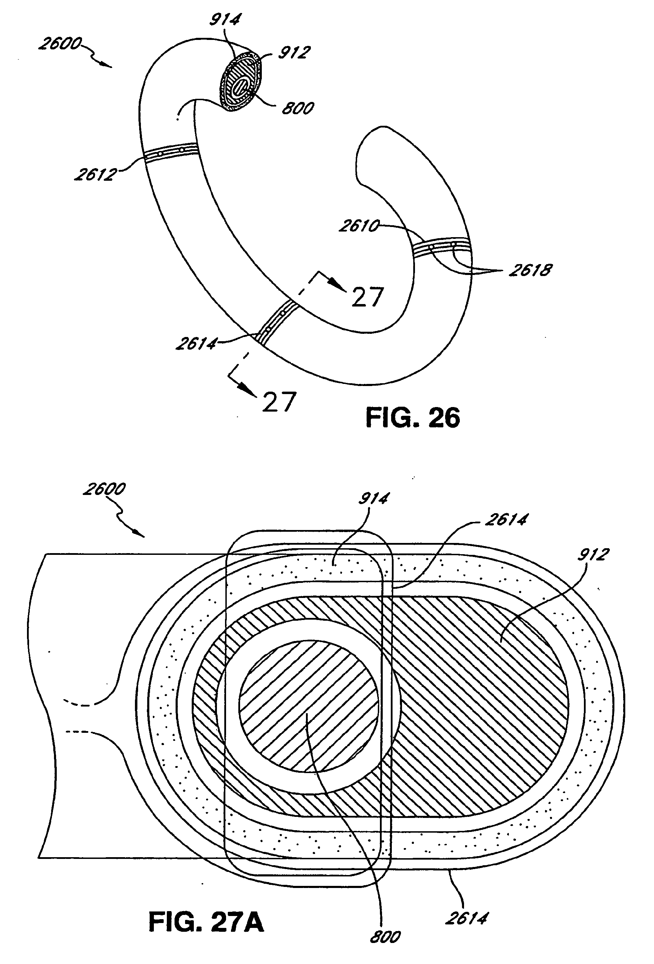

For example, FIG. 26 is a perspective view illustrating an annuloplasty ring 2600 comprising one or more thermal conductors 2610, 2612, 2614 not according to the invention. The annuloplasty ring 2600 further comprises a shape memory wire 800 covered by a flexible material 912 and a suturable material 914, such as the wire 800, the flexible material 912 and the suturable material 914 shown in FIG. 9A. As shown in FIG. 26, in certain examples, the shape memory wire 800 is offset from the center of the flexible material 912 to allow more room for sutures to pass through the flexible material 912 and suturable material 914 to attach the annuloplasty ring 2600 to a cardiac valve. In certain examples, the flexible material 912 and/or the suturable material 914 are thermally insulative. In certain such examples, the flexible material 912 comprises a thermally insulative material. Although the annuloplasty ring 2600 is shown in FIG. 26 as substantially C-shaped, an artisan will recognize from the disclosure herein that the one or more thermal conductors 2610, 2612, 2614 can also be used with other configurations including, for example, circular, D-shaped, or other curvilinear configurations.

In certain examples, the thermal conductors 2610, 2612, 2614 comprise a thin (e.g., having a thickness in a range between approximately 0.002 inches and approximately 0.015 inches) wire wrapped around the outside of the suturable material 914 and penetrating the suturable material 914 and the flexible material 912 at one or more locations 2618 so as to transfer externally applied heat energy to the shape memory wire 800. For example, FIGS. 27A-27C are transverse cross-sectional views of the annuloplasty ring 2600 schematically illustrating exemplary examples for conducting thermal energy to the shape memory wire 800. In the exemplary examples shown in FIG. 27A, the thermal conductor 2614 wraps around the suturable material 914 one or more times, penetrates the suturable material 914 and the flexible material 912, passes around the shape memory wire 800, and exits the flexible material 912 and the suturable material 914. In certain examples, the thermal conductor 2614 physically contacts the shape memory wire 800. However, in other examples, the thermal conductor 2614 does not physically contact the shape memory wire 800 but passes sufficiently close to the shape memory wire 800 so as to decrease the time required to activate the shape memory wire 800. Thus, the potential for thermal damage to surrounding tissue is reduced.

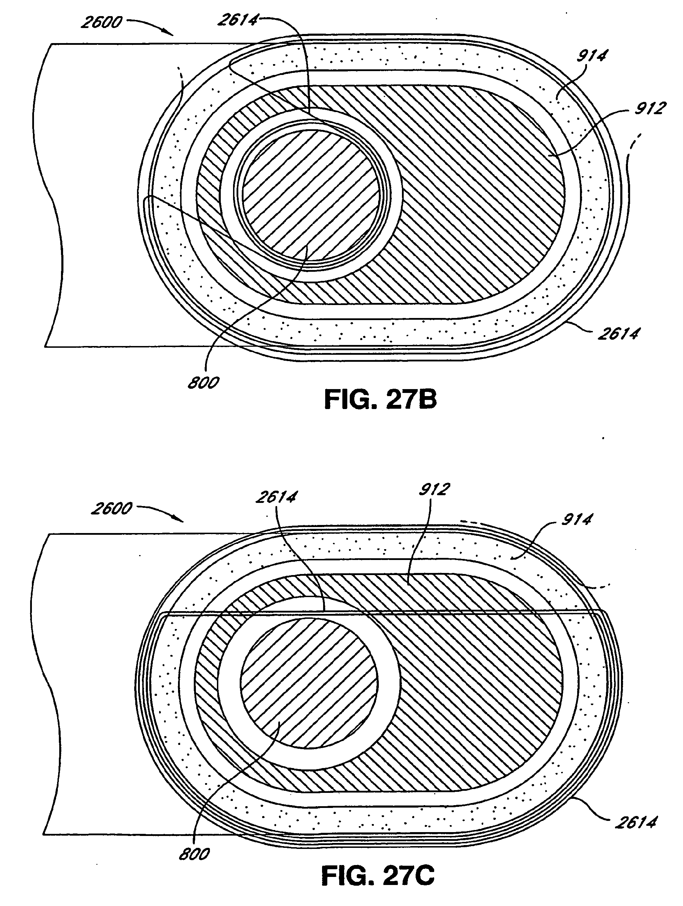

In the exemplary example shown in FIG. 27B, the thermal conductor 2614 wraps around the suturable material 914 one or more times, penetrates the suturable material 914 and the flexible material 912, passes around the shape memory wire 800 two or more times, and exits the flexible material 912 and the suturable material 914. By passing around the shape memory wire 800 two or more times, the thermal conductor 2614 concentrates more energy in the area of the shape memory wire 800 as compared to the exemplary embodiment shown in FIG. 27A. Again, the thermal conductor 2614 may or may not physically contact the shape memory wire 800.

In the exemplary examples shown in FIG. 27C, the thermal conductor 2614 wraps around the suturable material 914 one or more times and passes through the suturable material 914 and the flexible material 912 two or more times. Thus, portions of the thermal conductor 2614 are disposed proximate the shape memory wire 800 to transfer heat energy thereto. Again, the thermal conductor 2614 may or may not physically contact the shape memory wire 800. An artisan will recognize from the disclosure herein that one or more of the exemplary embodiments shown in FIGS. 27A-27C can be combined and that the thermal conductor 2614 can be configured to penetrate the suturable material 914 and the flexible material 912 in other ways in accordance with the invention so as to transfer heat to the shape memory wire 800.

Referring again to FIG. 26, in certain examples the locations of the thermal conductors 2610, 2612, 2614 are selected based at least in part on areas where energy will be applied to activate the shape memory wire 800. For example, in certain examples heat energy is applied percutaneously through a balloon catheter and the thermal conductors 2610, 2612, 2614 are disposed on the surface of the suturable material 914 in locations likely to make contact with the inflated balloon.

In addition, or in other examples, the thermal conductors 2610, 2612, 2614 are located to mark desired positions on the annuloplasty ring 2600. For example, the thermal conductors 2610, 2612, 2614 may be disposed at locations on the annuloplasty ring 2600 corresponding to commissures of heart valve leaflets, as discussed above with respect to FIG. 18. As another example, the thermal conductors 2610, 2612, 2614 can be used to align a percutaneous energy source, such as a heated balloon inserted through a catheter, with the annuloplasty ring 2600. In certain such examples the thermal conductors 2610, 2612, 2614 comprise radiopaque materials such as gold, copper or other imaging materials, as is known in the art.

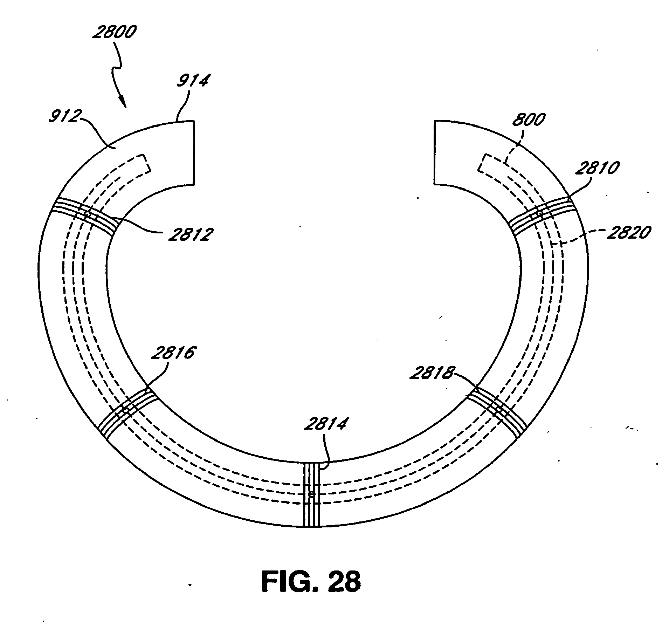

FIG. 28 is a schematic diagram of an annuloplasty ring 2800 not according to the invention comprising one or more thermal conductors 2810, 2812, 2814, 2816, 2818, such as the thermal conductors 2610, 2612, 2614 shown in FIG. 26. As schematically illustrated in FIG. 28, the annuloplasty ring 2800 further comprises a shape memory wire 800 covered by a flexible material 912 and a suturable material 914, such as the wire 800, the flexible material 912 and the suturable material 914 shown in FIG. 9A.

In certain examples, the shape memory wire 800 is not sufficiently thermally conductive so as to quickly transfer heat applied in the areas of the thermal conductors 2810, 2812, 2814, 2816, 2818. Thus, in certain such examples, the annuloplasty ring 2800 comprises a thermal conductor 2820 that runs along the length of the shape memory wire 800 so as to transfer heat to points of the shape memory wire 800 extending beyond or between the thermal conductors 2810, 2812, 2814, 2816, 2818. In certain examples, each of the thermal conductors 2810, 2812, 2814, 2816, 2818, comprise a separate thermally conductive wire configured to transfer heat to the thermal conductive wire 2820. However, in certain other examples, at least two of the thermal conductors 2810, 2812, 2814, 2816, 2818 and the thermal conductor 2820 comprise one continuous thermally conductive wire.

Thus, thermal energy can be quickly transferred to the annuloplasty ring 2600 or the annuloplasty ring 2800 to reduce the amount of energy required to activate the shape memory wire 800 and to reduce thermal damage to the patient's surrounding tissue.

### Magnetically Engageable Embodiments

FIG. 29A schematically illustrates a top view of an annuloplasty ring 2900 having a C-shaped configuration according to certain embodiments. The annuloplasty ring 2900 includes a continuous tubular member 2910 comprising a shape memory material that has a nominal inner transverse dimension that may contract or shrink upon the activation of the shape memory material by surgically or non-invasive applying energy thereto, as discussed above. The annuloplasty ring further comprises magnetic devices 2914 and 2916.

In certain embodiments, the annuloplasty ring 2900 comprises a shape memory wire covered by a flexible material and a suturable material, such as the wire 800, the flexible material 912 and the suturable material 914 shown in FIG. 9A.

The tubular member 2910 may comprise a homogeneous shape memory material, such as a shape memory polymer or a shape memory alloy including, for example, a ferromagnetic shape memory alloy. Alternatively, the tubular member 2910 may comprise two or more sections or zones of shape memory material having different temperature response curves, as discussed above with reference to FIG. 5. The shape memory response zones may be configured in order to achieve a desired configuration of the annuloplasty ring 2900 as a whole when in a contracted state, either fully contracted or partially contracted. In certain embodiments, the tubular member 2910 may enclose shape memory material. In certain embodiments, the tubular member 2910 may be made of at least a portion of shape memory material.

A suturable material (not shown), such as the suturable material 128 shown in FIG. 1, may be disposed about the annuloplasty ring 2900 and the annuloplasty ring 2900 may comprise or be coated with an energy absorption enhancement material 126, as discussed above.

The annuloplasty ring 2900 comprises one or more magnetic devices 2914 and 2916. In certain embodiments (not illustrated), the annuloplasty ring 2900 comprises one magnetic device, while in other embodiments, the annuloplasty ring 2900 comprises a plurality of magnetic devices. In certain embodiments, a magnetic device is located at only one end of the annuloplasty ring 2900, while in other embodiments, magnetic devices are located at both ends of the annuloplasty ring 2900. In certain embodiments, a magnetic device defines an end of the annuloplasty ring 2900, as shown in FIG. 29A. In certain embodiments, a magnetic device may be located between the ends of the annuloplasty ring 2900, as shown in FIG. 29B. In certain embodiments, the magnetic devices 2914 and 2916 may be disposed about the tubular member 2910. In certain embodiments, the magnetic devices 2914 and 2916 may be adjacent to an end of the tubular member 2910. In certain embodiments of the annuloplasty ring 2900 comprising more than one tubular member, magnetic devices may axially separate adjacent tubular members.

The term "magnetic" as used herein is a broad term and is used in its ordinary sense and includes, without limitation, any material that easily magnetizes, such as a material having atoms that orient their electron spins to conform to an external magnetic field. The magnetic devices 2914 and 2916 comprise materials exhibiting magnetic behavior or that may be magnetized by another magnet, including, but not limited to, ferromagnetism (including ferrimagnetism), diamagnetism and paramagnetism. Ferromagnetic materials include permanent magnets, ceramic magnets, electromagnetic materials, one or more polymer-bonded magnets, and isotropic and/or anisotropic materials, as discussed above. The magnetic devices 2914 and 2916 may also conduct thermal energy.

In certain embodiments, at least one of the magnetic devices 2914 and 2916 comprises a magnetic material. In certain embodiments, at least one of the magnetic devices 2914 or 2916 comprises an electromagnet that can be turned on or off. For example, the electromagnet may be turned on by providing an electric current to the magnet, and the electromagnet may be turned off by ceasing the flow of electric current.

In certain embodiments, one or more of the magnetic devices 2914 and 2916 can produce a force in a range between approximately 0,89 N (0.2 pounds of force) and approximately 8,9 N (2.0 pounds of force) at a distance of between one millimeter to ten millimeters, with a magnetic field in a range between approximately 100 Gauss and approximately 10,000 Gauss. In certain embodiments, a magnetic device may produce a force in a range between 0,44 N (0.1 pounds of force) and 13 N (3.0 pounds of force) at a distance between 0.5 millimeters and twenty millimeters. In certain embodiments, a magnetic device may produce a force in a range between 0,22 N (0.05 pounds of force) and 22 N (5.0 pounds of force). In certain embodiments, the magnetic field may have a range between approximately 100 gauss and approximately 700 gauss. In certain embodiments, the magnetic field may have a range between approximately 50 gauss and approximately 15,000 gauss.

The magnetic devices 2914 and 2916 may have any suitable configuration provided they are suitably biocompatible or covered with a biocompatible material for implantation in the human body, as discussed above.

In certain embodiments, the magnetic devices 2914 and 2916 are cylindrical shaped magnets having a positive pole and a negative pole. In certain such embodiments, one or more of the magnetic devices 2914 and 2916 has a diameter in a range between approximately 0.2 mm and approximately 0.4 mm, and a height in a range between approximately 0.2 mm and approximately 0.4 mm, which facilitates attachment of the magnetic devices 2914 and 2916 to the annuloplasty ring 2900. In other embodiments, the magnetic devices 2914 and 2916 may have other shapes. For example, in certain embodiments, one or more of the magnetic devices 2914 and 2916 can be in the shape of a rod, a sphere, a disk, a cube, a band, or the like. In certain embodiments, the magnets may have a different number of poles. In certain embodiments where the magnetic devices as disposed about the tubular member 2910, the magnetic devices may take the shape of a band around the tubular member.

In certain embodiments, the features, dimensions and materials of the annuloplasty ring 2900 are the same as or similar to the features, dimensions and materials of the annuloplasty ring 100 discussed above.

FIG. 29B schematically illustrates a top view of an annuloplasty ring 2902 having a C-shaped configuration according to certain embodiments. The annuloplasty ring 2902 includes a continuous tubular member 2910 comprising a shape memory material that has a nominal inner transverse dimension that may contract or shrink upon the activation of the shape memory material by surgically or non-invasive applying energy thereto, as discussed above. The annuloplasty ring further comprises magnetic devices 2914 and 2916 located between the ends of the annuloplasty ring 2902.

The tubular member 2910 may comprise a homogeneous shape memory material, such as a shape memory polymer or a shape memory alloy including, for example, a ferromagnetic shape memory alloy. Alternatively, the tubular member 2910 may comprise two or more sections or zones of shape memory material having different temperature response curves, as discussed above with reference to FIG. 5. The shape memory response zones may be configured in order to achieve a desired configuration of the annuloplasty ring 2902 as a whole when in a contracted state, either fully contracted or partially contracted. In certain embodiments, the tubular member 2910 may enclose shape memory material. In certain embodiments, the tubular member 2910 may be made of at least a portion of shape memory material.

A suturable material (not shown), such as the suturable material 128 shown in FIG. 1, may be disposed about the annuloplasty ring 2900 and the annuloplasty ring 2902 may comprise or be coated with an energy absorption enhancement material 126, as discussed above.

The annuloplasty ring 2902 comprises one or more magnetic devices 2914 and 2916 located between the ends of the annuloplasty ring 2902. The magnetic devices 2914 and 2916 of the annuloplasty ring 2902 comprise one or more magnetic bands disposed about the tubular member 2910. In certain embodiments, In certain embodiments, the annuloplasty ring 2902 may comprise more than one tubular member axially separated by magnetic devices. In certain embodiments, the magnetic bands 2914 and 2916 are located at or near the ends of the annuloplasty ring 2902. In certain embodiments, the magnetic bands 2914 and 2916 are located substantially near the midpoint between the both ends of the annuloplasty ring 2902. In certain embodiments, the one or more magnetic bands 2914 and 2916 are located at various points along the annuloplasty ring 2902. In certain embodiments (not illustrated), the annuloplasty ring 2902 comprises one magnetic device, while in other embodiments, the annuloplasty ring 2902 comprises a plurality of magnetic devices 2914 and 2916.

In certain embodiments, the outside surface of the magnetic devices 2914 and 2916 is coated with a thin coating of biocompatible material as discussed above, including fluorinated ethylene propylene (FEP), polyether ether kythane (PEEK®), polycarbonate, polypropylene, high density polyethylene (HDPE), modified ethylene-tetrafluoroethylene copolymer (ETFE), polyethylene terephthalate polyester (PET-P), polyimide, nylon 6/12, and acrylonitrile/butadiene/styrene resin (ABS). In other embodiments, the suturable material comprises a biological material such as bovine or equine pericardium, homograft, patient graft, or cell-seeded tissue. An outer layer of rigid material, as described above, can also be used.

In certain embodiments, the features, dimensions and materials of the annuloplasty ring 2902 are the same as or similar to the features, dimensions and materials of the annuloplasty ring 100 discussed above.

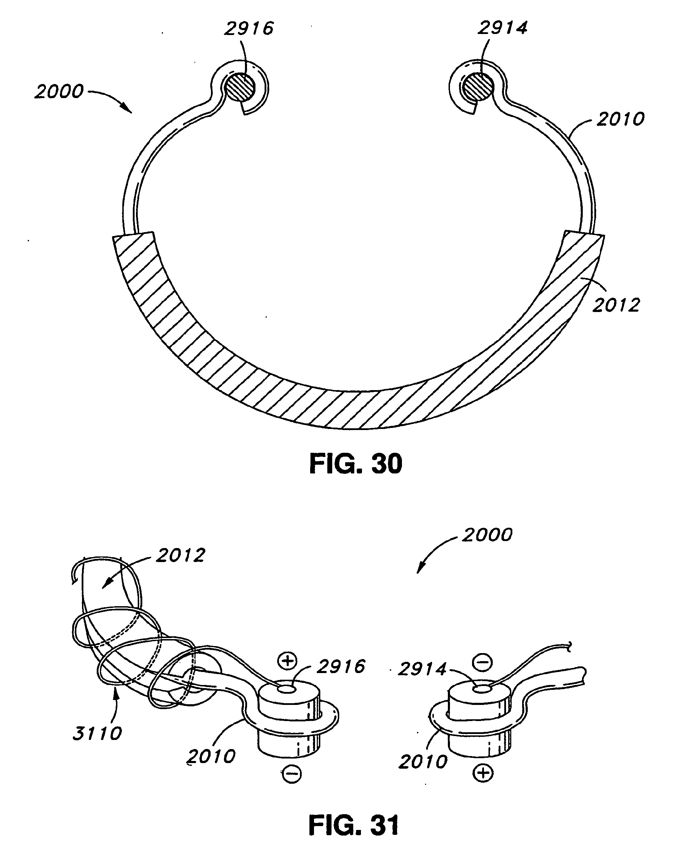

FIG. 30 schematically illustrates one embodiment of the body member 2000 shown in FIG.20. The body member 2000 comprises a wire 2010, a shape memory tube 2012, and magnetic devices 2914 and 2916. Although not shown, in certain embodiments, the body member 2000 is covered by a flexible material such as silicone rubber and a suturable material such as woven polyester cloth, Dacron@, woven velour, polyurethane, polytetrafluoroethylene (PTFE), heparin-coated fabric, or other biocompatible material, as discussed above. Although also not shown, in certain embodiments, the body member 2000 comprises an energy absorption enhancement material, as discussed above.

In certain embodiments, the body member 2000 is configured to enter multiple configurations when activated, as discussed above with reference to FIGS. 20A and 20B.

The body member 2000 comprises one or more magnetic devices 2914 and 2916. In certain embodiments (not illustrated), the body member 2000 comprises one magnetic device, while in other embodiments, the body member 2000 comprises a plurality of magnetic devices.

In certain embodiments, the wire 2010 attaches the magnetic devices 2914, 2916 to the first and second ends, respectively, of the body member 2000. In certain embodiments, the wire 2010 wraps around the magnetic devices 2914 and 2916 in a semicircular, or hook-shaped fashion. In other embodiments, the wire 2010 wraps around the magnetic devices 2914 and 2916 in a loop, as illustrated in FIG. 30. For example, each end of the wire 2010 may form a circle shape around the magnetic devices 2914 and 2916. In certain embodiments, the wire 2010 wraps around the magnetic devices 2914 and 2916 in a series of loops. For example, the wire 2010 may form a spiral or helical shape around the magnetic devices 2914 and 2916. In other embodiments, the wire 2010 may form other shapes when coupling with the magnetic devices 2914 and 2916. In certain embodiments, the wire 2010 may couple to the magnetic devices 2914 and 2916 using other methods.

Although the embodiments 2900, 2902, and 2000 of FIGS. 29A, 29B, and 30, respectively, are illustrated in a substantially C-shaped configuration, in other embodiments the body member can be selected from a variety of shapes including, for example, ring shaped or D-shaped, as shown with regard to other embodiments discussed above. In certain embodiments, the features, dimensions and materials of embodiments 2900 and 2902 are the same as or similar to the features, dimensions and materials of the annuloplasty ring 2000 discussed above.

FIG. 31 schematically illustrates in more detail the first and second ends of the body member 2000 as illustrated in FIG. 30. As shown in FIG. 31, the body member 2000 comprises the wire 2010, the shape memory tube 2012, and the magnetic devices 2914 and 2916. As discussed above, in certain embodiments the magnetic devices 2914 and 2916 each comprise a positive pole and a negative pole. In the embodiment illustrated in FIG. 31, the first magnetic device 2914 is orientated such that its negative pole is on a top surface of the magnetic device 2914, and the second magnetic device 2916 is oriented such that its positive pole is on a top surface of the magnetic device 2916. As discussed below, in certain embodiments, opposite poles of the respective magnetic devices facing one direction advantageously improves alignment with one or more catheters. Thus, the body member 2000 can engage the annuloplasty ring 1826 in a desired orientation and with improved contact to increase contact surface area and contact holding force.

In other embodiments, the magnetic devices can have different orientations. In certain other embodiments, the first magnetic device 2914 can be orientated such that its positive pole is on its top surface, and the second magnetic device 2916 can be oriented such that its negative pole is on its top surface. In certain other embodiments, the first and second magnetic devices 2914 and 2916 can be orientated such that their positive poles are on their respective top surfaces. In certain other embodiments, the first and second magnetic devices 2914 and 2916 can be orientated such that their negative poles are on their respective top surfaces.

The body member 2000 further comprises one or more energy conductors 3110, according to certain embodiments of the invention. In certain embodiments, the conductors 3110 comprise thermal conductors. In certain embodiments, the conductors are configured to conduct other forms of energy, such as radio frequency (RF) energy, x-ray energy, microwave energy, ultrasonic energy such as focused ultrasound, high intensity focused ultrasound (HIFU) energy, light energy, electric field energy, magnetic field energy, combinations of the foregoing, or the like.

In certain embodiments, the conductors 3110 comprise a thin wire wrapped around the outside of the shape memory tube 2012 to transfer externally applied heat energy to the shape memory tube 2012. For example, the wire may have a thickness in a range between approximately 0,05 mm (0.002 inches) and approximately 0,38 mm (0.015 inches). In certain embodiments, the wire may have a thickness in a range between approximately 0,025 mm (0.001 inches) and approximately 2,0 mm (0.080 inches). In certain embodiments, the wire may have a thickness in a range between approximately 0,013 mm (0.0005 inches) and approximately 2,5 mm (0.1 inches). In certain embodiments, the conductors 3110 are attached to one or both of the magnetic devices 2914 and 2916, In certain embodiments, the conductors 3110 may comprise a casing wrapped around the outside of the shape memory tube 2012. In certain embodiments, the conductors 3110 may comprise platinum coated copper, titanium, tantalum, stainless steel, gold, their combinations, or the like, as discussed above.

In certain embodiments, the conductors 3110 comprise heating wire 3110, which that generate heat when activated by an electrically conductive element, such as an electrical source or a heat source. For example, the heating wire 3110 may be activated by a catheter comprising a contact portion that may be heated, as discussed below.

In certain embodiments, the heating wire 3110 comprises a thin wire, such as a nickel-chromium resistance wire or iron-chrome-aluminum wire, wrapped around the outside of the shape memory tube 2012. In certain embodiments, the wire may have a thickness in a range between approximately 0,05mm (0.002 inches) and approximately 0,38 mm (0.015 inches). In certain embodiments, the wire may have a thickness in a range between approximately 0,025 mm (0.001 inches) and approximately 2,0 mm (0.080 inches). In certain embodiments, the wire may have a thickness in a range between approximately 0,013 mm (0.0005 inches) and approximately 2,5 mm (0.1 inches). In certain embodiments, the heating wire 3110 is encased in a biocompatible material as described above.

In certain embodiments, the heating wire 3110 is attached to one or both of the magnetic devices 2914 and 2916. The wire 3110 transfers heat energy generated or conducted by the wire 3110 to the shape memory tube 2012. For example, if the wire 3110 is heated using an external device, a quantity of the heat from the wire 3110 may pass to the shape memory tube 2012 through a contact point between the two objects in order to heat the shape memory tube 2012 to an austensite temperature.

In certain embodiments, the conductor 3110, such as the heating wire, may not physically contact with the shape memory tube 2012. In certain embodiments, the conductor 3110 can pass around and/or through the covering of an annuloplasty ring, such as annuloplasty rings 1826 or 2600, to transfer heat or electric current to the shape memory tube 2012. In certain embodiments, the conductor 3110 passes sufficiently close to the shape memory tube 2012 so as to decrease the time required to activate the shape memory tube 2012. Thus, the potential for damage to surrounding tissue is reduced.

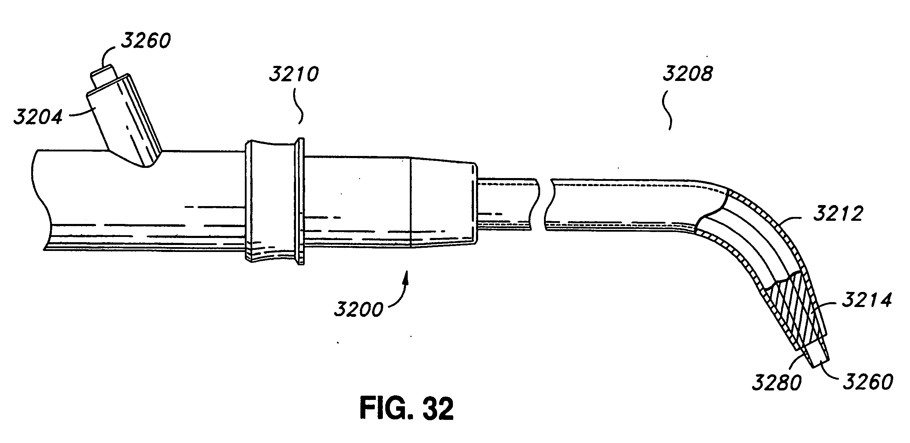

FIG. 32 is a perspective view of a magnetic tipped catheter 3200 configured to deliver energy to an implant according to certain embodiments. The energy source may include, for example, radio frequency (RF) energy, x-ray energy, microwave energy, ultrasonic energy such as focused ultrasound, high intensity focused ultrasound (HIFU) energy, light energy, electric field energy, magnetic field energy, combinations of the foregoing, or the like.

The catheter 3200 comprises a catheter body portion 3210 at a proximal end of the catheter 3200 and a distal portion 3208 at a distal end of the catheter 3200. The distal portion 3208 comprises a flexible tip portion 3212, which itself comprises a magnetic tip 3214 configured to emanate a magnetic field. For example, the magnetic tip illustrated has a positive pole and a negative pole. In certain embodiments, the flexible tip portion 3212 may be made using flexible material, as described above.

In certain embodiments, the magnetic tip 3214 comprises a ferromagnetic material, as described above. In certain embodiments, the positive pole of the magnetic tip 3214 is at the distal end of the catheter 3200. In certain other embodiments, the negative pole of the magnetic tip 3214 is at the distal end of the catheter 3200. The magnetic tip 3214 can be adhered, welded, soldered, glued, or otherwise incorporated into the distal portion 3208 as desired. In certain embodiments, the magnetic tip 3214 may comprise a plurality of magnetic bands. The magnetic bands can be evenly or unevenly spaced along the length of the distal portion 3208. The magnetic devices 2914 and 2916 of the implantable device 2000 can be positioned at any suitable location to aid in the positioning of the distal portion 3208 of the catheter 3200 relative to the implant 2000.

The body portion 3210 includes a side arm 3204 through which catheter ports 3260 may be accessed. In certain embodiments, the catheter 3200 may contain one port, while in other embodiments the catheter 3200 may contain more than one port. In certain embodiments, the catheter 3200 may not contain any ports. A catheter port 3260 may be used to facilitate the insertion and pushing of instruments or objects, such as heated fluid or a heated fluid balloon, or fiber optic elements through the catheter body 2604 so as to deliver them to heart tissue.

Heart tissue may be accessed during an operation by various techniques and procedures so that the implantable device 2000 can be activated. For example, minimal invasive surgery techniques, laparoscopic procedures, and/or open surgical procedures can provide a convenient access path to the chambers of the heart for delivering energy using the magnetic tipped catheter 3200. In some embodiments, access to the heart can be provided through the chest of a patient, and may include, without limitation, conventional transthoracic surgical approaches, open and semi-open heart procedures, and port access techniques. Such surgical access and procedures preferably can utilize conventional surgical instruments for access and performing surgical procedures on the heart, for example, retractors, rib spreaders, trocars, laparoscopic instruments, forceps, cannulas, staplers, and the like. The implant 2000 can be activated in conjunction with another surgical procedure that provides access (e.g., mitral valve repair, bypass surgical procedures, etc.).

Generally, in an embodiment intended for access through the femoral vein and delivery to the left atrium, the catheter 3200 can have a length within the range of from about 50 cm to about 150 cm, and a diameter of generally no more than about 1,7 mm (5 French), 3,3 mm (10 French), or 5 mm (15 French). Those skilled in the art recognize that the catheter system can be configured and sized for various methods of activating the implantable device, as described below. The catheter 3200 can be sized and configured so that it can be delivered using, for example, conventional transthoracic surgical, minimally invasive, or port access approaches. In view of the present disclosure, further dimensions and physical characteristics of catheters for navigation to particular sites within the body are well understood in the art.

In embodiments where the catheter 3200 is delivered percutaneously into the heart, a guiding sheath can be placed in the vasculature system of the patient and used to guide the catheter 3200 to a desired deployment site.

In some embodiments, a guide wire is used to gain access through the superior or inferior vena cava, for example, through groin access for delivery through the inferior vena cava. The guiding sheath can be advanced over the guide wire and into the inferior vena cava. The distal end of the guiding sheath can be passed through the right atrium and towards the septum. Once the distal end of the guiding sheath is positioned proximate to the septum, a needle or piercing member is preferably advanced through the guiding sheath and used to puncture the fossa ovalis or other portion of the septum. In some embodiments, the guiding sheath is dimensioned and sized to pass through the fossa ovalis without requiring a puncturing device. That is, the guiding sheath can pass through the natural anatomical structure of the fossa ovalis into the left atrium.

The guiding sheath can be positioned through the inferior vena cava through the right atrium and a septal hole. When the guiding sheath is positioned within the heart, the catheter 3200 can be advanced distally through the guiding sheath. As the catheter 3200 is advanced through the guiding sheath, the distal portion 3208 is somewhat straight. Thus, the distal portion 3208 can be delivered through a low profile delivery sheath and can flex as it is advanced distally.

The catheter system 3200 can be advanced until the distal portion 3208 passes out of an opening of the guiding sheath. Preferably, the distal portion 3208 is in a generally collapsed state (e.g., a deflated state) as it is delivered through the guiding sheath for a low profile configuration.

As the distal portion 3208 passes out of the opening of the guiding sheath, the distal portion can assume its at-rest configuration. The distal portion 3208 assumes a somewhat curved configuration as it extends out of the opening. Of course, the catheter system 3200 can be twisted and rotated within the guiding sheath to position the distal portion 3208 comprising the magnetic tip 3214.

In some embodiments, the magnetic tip 3214 of the distal portion 3208 can be an atraumatic tip that is configured to slide through the lumen of the delivery sheath. The atraumatic tip 3214 can limit or prevent significant damage to the inner tissue of the heart.

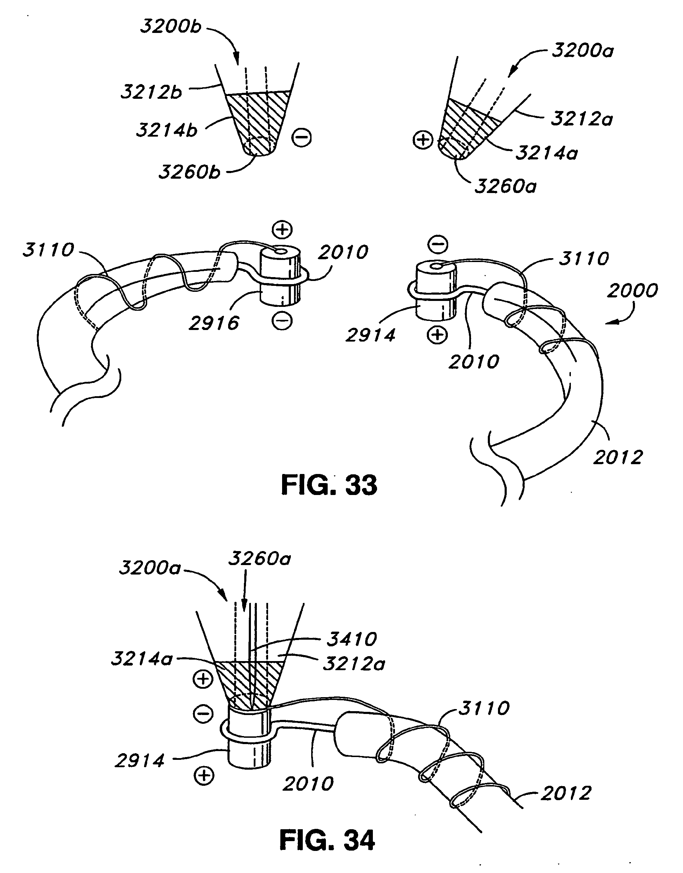

FIG. 33 schematically illustrates one exemplary embodiment of aligning one or more catheters with an implant, such as body member 2000 of FIG. 30, within the human body. Although the embodiment of body member 2000 is illustrated, other implants, including annuloplasty rings 2900 and 2902, may also be used.

The body member 2000 comprises the first magnetic device 2914 attached to the first end of the body member 2000, the second magnetic device 2916 attached to the second end of the body member 2000, and the conductor 3110. FIG. 33 also illustrates a two catheters 3200a and 3200b, such as the catheter 3200 of FIG. 32. The first catheter 3200a comprises a first flexible tip portion 3212a and a first magnetic tip 3214a, and a second catheter 3200b comprising a second flexible tip portion 3212b and a second magnetic tip 3214b.

After the body member 2000 has been implanted into a patient, a first catheter 3200a is inserted into the patient and guided to the annuloplasty implant area, as discussed above. The pole on the outer surface of the first magnetic tip 3214a and pole on the top surface of the first magnet device 2914 are of opposite polarities such that the magnetic tip 3214a and the first magnetic device 2914 produce a mutually attractive force. In certain embodiments, the magnetic tip 3214a and the first magnet device 2914 magnetically attach to each other, thus allowing the catheter 3200a to align with and attach to the implanted body member.

The illustrated embodiment shows the first magnetic tip 3214a having a positive pole on its outer surface and the first magnetic device 2914 having a negative pole on its top surface. In certain other embodiments, the first magnetic tip 3214a has a negative pole on its outer surface and the first magnetic device 2914 has a positive pole on its top surface such that the magnetic tip 3214a and the first magnetic device 2914 produce a mutually attractive force.

In certain other embodiments, a second catheter 3200b is also inserted into the patient and guided to the annuloplasty implant area. The poles of the magnetic tip 3214b and the second magnetic device 2916 are of opposite polarities such that the magnetic tip 3214b and the first magnetic device 2916 produce a mutually attractive force. In certain embodiments, the magnetic tip 3214b and the second magnet device 2914 magnetically attach to each other. Thus, both catheters 3200a and 3200b can be aligned with and attached to the implanted annuloplasty ring.

The illustrated embodiment shows the second magnetic tip 3214b having a negative pole on its outer surface and the second magnetic device 2916 having a positive pole on its top surface. In certain other embodiments, the second magnetic tip 3214b has a positive pole on its outer surface and the second magnetic device 2916 has a negative pole on its top surface such that the magnetic tip 3214b and the second magnetic device 2916 produce a mutually attractive force.

After the magnetic tip 3214a magnetically attaches to the magnetic device 2914, as shown in FIG. 34, the distal portion 3208 of the catheter 3200a can deliver energy to the conductor 3110. The delivered energy may include, for example, thermal energy, radio frequency (RF) energy, x-ray energy, microwave energy, ultrasonic energy such as focused ultrasound, high intensity focused ultrasound (HIFU) energy, light energy, electric field energy, magnetic field energy, combinations of the foregoing, or the like. In certain embodiments, energy may be delivered to the implant using an energy transfer module, such as a magnet, a conductor, a wire, or a needle-type "pointer" or any other suitable structure in thermal or electrical communication with the implant. In certain embodiments, thermal energy may be delivered to the implant via the conductor 3110. In certain embodiments, thermal energy may be delivered to the implant via the implant's magnetic devices 2914 and 2916. In certain embodiments, the energy may be delivered to the implant via another surface of the implant.

In certain embodiments, thermal energy may be delivered. In the embodiment illustrated in FIG. 34, thermal energy may be delivered to the conductor 3110 via the catheter 3200 using a needle type of pointer 3410 inserted through the catheter 3200a to contact the conductor 3110. In certain embodiments, the pointer 3410 may be inserted through a port 3260 of the catheter 3200. In certain embodiments not illustrated, the pointer 3410 may be external to and/or travel alongside an exterior surface of the catheter 3200a. In certain embodiments, the pointer 3410 conducts thermal energy from an external source to the conductor 3110. The conductor 3110 in turn, transfers the thermal energy to the shape memory tube 2012, which causes the annuloplasty ring to alter its shape. In certain embodiments, the pointer 3410 heats the magnetic tip 3214 of the catheter 3200, which, when in contact with the conductor 3110, heats the conductor 3110.

In embodiments where catheter 3200 activates an implant by sending activation energy to a magnetic device of the implant, energy may be delivered to either one or several magnetic devices simultaneously. In embodiments where the implant is composed of a plurality of shape memory segments, the catheter 3200 may activate the implant by sending activation energy to one or several shape memory segments simultaneously.

In certain other embodiments, after the first and second catheters 3200a, 3200b are magnetically attached to the first and the second magnetic devices 2914, 2916, respectively, a first pointer 3410 can be inserted through the catheter 3200a, and a second pointer 3410 can be inserted through the catheter 3200b. The first pointer 3410 contacts the heating wire 3110 at the first magnetic device 2914, and the second pointer 3420 contacts the heating wire 3110 at the second magnetic device 2916. In certain embodiments, a current passing from the first pointer 3410 to the second pointer 3410 can provide a circuit through the heating wire 3110, which causes the heating wire 3110 to generate heat. Thus, the heating wire 3110 transfers thermal energy to the shape memory tube 2012, which causes the annuloplasty ring to alter its shape.

In certain embodiments, an electric current may pass through the catheter 2900 which heats the end portion 3280 of the flexible tip 3212. In certain embodiments, the electric current may heat the magnetic tip 3214. The catheter 3200 may thus be used to apply energy to an implant as described above, such as implants 2000, 2900 and 2902 comprising magnetic devices described above.

In certain embodiments of the catheter 3200, media, such as a fluid like water or saline, can be injected through the catheter port 3260 and through the body portion 3210 to the distal portion's flexible tip 3212. The media may or may not be heated. Preferably, the media is heated to a threshold or target temperature before being delivered to the distal portion 3208. The media can flow through the a port 3260 of the catheter and heat the flexible tip portion 3212 of the catheter 3200. For example, the flexible tip portion 3212 may contain a balloon member (not illustrated) that is inflated by the media and heated as the heated media fills the distal portion 3208. The heat from the distal portion 3208 can be transferred to the body member 2000, preferably being transferred at least until the body member 2000 is activated, thereby changing the shape of the implantable device 2900.

After the body member 2000 has been activated, the catheter 3200 can be retracted or moved proximally relative to the guide sheath. As the catheter system 3200 is pulled proximally through the guide sheath, the distal portion is straightened and slid through the opening and into the guide sheath. The catheter 3200 and the guide sheath can be withdrawn from the vasculature, preferably withdrawn without damaging the vasculature tissue.

While certain embodiments of the inventions have been described, these embodiments have been presented by way of example only, and are not intended to limit the scope of the inventions. Indeed, the novel methods and systems described herein may be embodied in a variety of other forms. Furthermore, various omissions, substitutions and changes in the form of the methods and systems described herein may be made without departing from the spirit of the inventions. For example, certain annuloplasty rings discussed herein can be flexible, semi-rigid, or rigid. The accompanying claims and their equivalents are intended to cover such forms or modifications as would fall within the scope and spirit of the invention.

## Claims

1. An annuloplasty activation system for percutaneously activating an adjustable annuloplasty device, the system comprising:
an annuloplasty device (2000,2900,2902);
an elongate member (3200) having a proximal end (3210) and distal end (3208), the distal end configured to be inserted percutaneously into a vein of a patient, further configured to be inserted across a fossa ovalis in an interatrial septum of a patient, and further configured to be inserted into the left atrium of the patient while the proximal end is located outside the patient's body; and
a distal element (3214) at the distal end (3208) of the elongate member (3200),
**characterized in that** the distal element (3214) is configured to emanate a magnetic field within the left atrium, is configured to magnetically attach to the annuloplasty device (2000, 2900, 2902) and further is configured to deliver energy through the distal end (3208) to the annuloplasty device (2000);
wherein the distal element (3214) comprises at least one of a permanent magnet and an electromagnet, the electromagnet being activatable from outside the patient's body; and
wherein the adjustable annuloplasty device configured to be adjusted by applying an activation energy through the distal element to activate a shape memory material in the adjustable annuloplasty device and cause it to change to a memorized shape.

2. The annuloplasty activation system of Claim 1, wherein the annuloplasty device (2000, 2900, 2902) comprises:
a body member having a proximal end, a distal end, and a length extending therebetween, the body member configured to be implanted within a patient's heart at or near a base of a heart valve;
wherein the body member comprises:
a first portion, comprising the shape memory material and being transformable from a first configuration to a second configuration in response to the activation energy; and
a second portion coupled to the first portion, the second portion comprising a magnetic material (2914, 2916) that is responsive to the magnetic field; and
wherein, when in position at or near the base of the heart valve and when the body member transforms from the first configuration to the second configuration, the body member is adapted to reshape a tissue of the heart so as to exert a force on the heart valve base.

3. The annuloplasty activation system of Claim 1, wherein the distal element (3214) is further configured to deliver electric field energy, thermal energy, magnetic field energy, radio frequency energy, x-ray energy, microwave energy, ultrasonic energy, light energy, or combinations of the foregoing.

4. The annuloplasty activation system of Claim 1, wherein the distal end (3208) comprises a flexible portion (3212).

5. The annuloplasty activation system of Claim 1, wherein the distal element (3214) comprises a plurality of magnetic bands.

6. The annuloplasty activation system of Claim 1, wherein the elongate member (3200) comprises a port (3260) at the proximal end (3210) in fluid communication with the distal end (3208).

7. The annuloplasty activation system of Claim 1, wherein the elongate member (3200) is configured to deliver energy to the annuloplasty device (2000).

8. The annuloplasty activation system of Claim 2, wherein the magnetic material produces a force between approximately 0.89 N (0.2 pounds) of force and approximately 8.9 N (2.0 pounds) of force, at a distance of between 1 millimeter to 10 millimeters.

9. The annuloplasty activation system of Claim 8, wherein the magnetic material produces a magnetic field between approximately 100 Gauss and approximately 10,000 Gauss.

10. The annuloplasty activation system of Claim 2, wherein the magnetic material produces a force in a range between 0.45 N (0.1 pounds) of force and 13.35 N (3.0 pounds) of force at a distance between 0.5 millimeters and twenty millimeters and has a range between approximately 100 Gauss and approximately 700 Gauss.

11. The annuloplasty activation system of Claim 2, wherein the magnetic material comprises cylindrical shaped magnets.

12. The annuloplasty activation system of Claim 11, wherein the magnets have a diameter between approximately 0.2 mm and approximately 0.4 mm, and a height between approximately 0.2 mm and approximately 0.4 mm.

13. The annuloplasty activation system of Claim 2, wherein the magnetic material comprises a rod shape, a spherical shape, a disk shape, a cubic shape, or a band shape.

14. The annuloplasty activation system of Claim 2, wherein the annuloplasty device (2000) is ring shaped.

15. The annuloplasty activation system of Claim 2, wherein the annuloplasty device (2000) is C-shaped.

16. The annuloplasty activation system of Claim 2, wherein the annuloplasty device (2000) is D-shaped.

## Patentansprüche

1. Annuloplastie-Aktivierungssystem zum perkutanen Aktivieren einer einstellbaren Annuloplastievorrichtung, wobei das System umfasst:
eine Annuloplastievorrichtung (2000, 2900, 2902);
ein längliches Element (3200) mit einem proximalen Ende (3210) und einem distalen Ende (3208), wobei das distale Ende aufgebaut ist, um perkutan in eine Ader eines Patienten eingeführt zu werden, wobei es ferner aufgebaut ist, um quer durch eine Fossa Ovalis in einem interarterialen Septum eines Patienten eingeführt zu werden, und ferner aufgebaut ist, um in das linke Atrium des Patienten eingeführt zu werden, während das proximale Ende sich außerhalb des Körpers des Patienten befindet; und
ein distales Element (3214) an dem distalen Ende (3208) des länglichen Elements (3200),
**dadurch gekennzeichnet, dass** das distale Element (3214) aufgebaut ist, um ein magnetisches Feld innerhalb des linken Atriums auszustrahlen, aufgebaut ist, um die Annuloplastievorrichtung (2000, 2900, 2902) magnetisch zu befestigen, und ferner aufgebaut ist, um Energie durch das distale Ende (3208) an die Annuloplastievor richtung (2000) abzugeben;
wobei das distale Element (3214) einen Permanentmagneten und/oder einen Elektromagneten umfasst, wobei der Elektromagnet von außerhalb des Körpers des Patienten aktivierbar bzw. ansteuerbar ist; und
wobei die einstellbare Aunnuloplastievorrichiung aufgebaut ist, um durch Anwenden einer Aktivierungsenergie durch das distale Element ein Formgedächtnismaterial in der einstellbaren Annuloplastievorrichtung zu aktivieren und zu bewirken, dass es sich in eine ins Gedächtnis eingeprägte Form ändert.

2. Annuloplastie-Aktivierungssystem nach Anspruch 1, wobei die Annuloplastievorrichtung (2000, 2900, 2902) umfasst:
ein Rumpfelement mit einem proximalen Ende, einem distalen Ende und einer Länge, die sich dazwischen erstreckt, wobei das Rumpfelement aufgebaut ist, um innerhalb eines Herzens eines Patienten bei oder nahe einer Herzklappenbasis implantiert zu werden,
wobei das Rumpfelement umfasst:
einen ersten Abschnitt, der das Formgedächtnismaterial umfasst und ansprechend auf die Aktivierungsenergie von einem ersten Aufbau in einen zweiten Aufbau umwandelbar ist; und
einen zweiten Abschnitt, der mit dem ersten Abschnitt gekoppelt ist, wobei der zweite Abschnitt ein magnetisches Material (2914, 2916) umfasst, das auf das Magnetfeld anspricht; und
wobei das Rumpfelement, wenn es an seiner Position bei oder nahe der Herzklappenbasis ist und das Rumpfelement sich von dem ersten Aufbau in den zweiten Aufbau umwandelt, geeignet ist, ein Gewebe des Herzens umzuformen, um eine Kraft auf die Herzklappenbasis auszuüben.

3. Annuloplastie-Aktivierungssystem nach Anspruch 1, wobei das distale Element (3214) ferner aufgebaut ist, um elektrische Feldenergie, Wärmeenergie, magnetische Feldenergie, Funkfrequenzenergie, Röntgenstrahlenenergie, Mikrowellenenergie, Ultraschallenergie, Lichtenergie oder Kombinationen der vorangehenden abzugeben.

4. Annulcplastie-Aktivierungssystem nach Anspruch 1, wobei das distale Ende (3208) einen flexiblen Abschnitt (3212) umfasst.

5. Annuloplastie-Aktivierungssystem nach Anspruch 1, wobei das distale Element (3214) eine Vielzahl von magnetischen Bändern umfasst.

6. Annuloplastie-Aktivierungssystem nach Anspruch 1, wobei das längliche Element (3200) eine Mündung (3260) an dem proximalen Ende (3210) in Fluidverbindung mit dem distalen Ende (3208) umfasst.

7. Annuioplastie-Aktivierungssystem nach Anspruch 1, wobei das längliche Element (3200) aufgebaut ist, um Energie an die Annuloplastievorrichtung (2000) abzugeben.

8. Annuloplastie-Aktivierungssystem nach Anspruch 2, wobei das magnetische Material in einer Entfernung zwischen 1 Millimeter und 10 Millimetern eine Kraft zwischen etwa 0,89 N (0,2 Pfund) Kraft und etwa 8,9 N (2,0 Pfund) Kraft erzeugt.

9. Annuloplastie-Aktivierungssystem nach Anspruch 8, wobei das magnetische Material ein Magnetfeld zwischen etwa 100 Gauß und etwa 10000 Gauß erzeugt.

10. Annuloplastie-Aktivierungssystem nach Anspruch 2, wobei das magnetische Material in einer Entfernung zwischen 0,5 Millimeter und zwanzig Millimetern eine Kraft in einem Bereich zwischen etwa 0,45 N (0,1 Pfund) Kraft und etwa 13,35 N (3,0 Pfund) Kraft erzeugt und einen Bereich von etwa 200 Gauß bis etwa 700 Gauß hat.

11. Annuloplastie-Aktivierungssystem nach Anspruch 2, wobei das magnetische Material zylindrisch geformte Magnete umfasst.

12. Annuloplastie-Aktivierungssystem nach Anspruch 11, wobei die Magnete einen Durchmesser von etwa 0,2 mm und etwa 04 mm und eine Höhe zwischen etwa 0,2 mm und etwa 0,4 mm haben.

13. Annuloplastie-Aktivierungssystem nach Anspruch 2, wobei das magnetische Material eine Stangenform, eine Kugelform, eine Scheibenform, eine Würfelform oder eine Bandform umfasst.

14. Annuloplastie-Aktivierungssystem nach Anspruch 2, wobei die Annuloplastievorrichtung (2000) ringförmig ist.

15. Annuloplastie-Aktivierungssystem nach Anspruch 2, wobei die Annuloplastievorrichtung (2000) C-förmig ist.

16. Annuloplastie-Aktivierungssystem nach Anspruch 2, wobei die Annuloplastievorrichtung (2000) D-förmig ist.

## Revendications

1. Système d'activation d'annuloplastie pour activer par voie percutanée un dispositif d'annuloplastie ajustable, le système comprenant :
un dispositif d'annuloplastie (2000, 2900, 2902) ;
un élément allongé (3200) ayant une extrémité proximale (3210) et une extrémité distale (3208), l'extrémité distale étant configurée pour être insérée par voie percutanée dans la veine d'un patient, ultérieurement configurée pour être insérée à travers une fossette semi-ovoïde dans une cloison inter-auriculaire d'un patient, et ultérieurement configurée pour être insérée dans l'oreillette gauche du patient, tandis que l'extrémité proximale est située hors du corps du patient ; et
un élément distal (3214) à l'extrémité distale (3208 de l'élément allongé (3200), **caractérisé en ce que** l'élément distal (3214) est configuré de façon à émettre un champ magnétique dans l'oreillette gauche, pour fixer magnétiquement le dispositif d'annuloplastie (2000, 2900, 2902) et est ultérieurement configuré pour fournir de l'énergie à travers l'extrémité distale (3208) au dispositif d'annuloplastie (2000) ;
dans lequel l'élément distal (3214) comprend au moins un aimant permanent ou un électroaimant, l'électroaimant pouvant être activé depuis l'extérieur du corps du patient ; et
dans lequel le dispositif d'annuloplastie ajustable est configuré pour être réglé en appliquant une énergie d'activation à travers l'élément distal, afin d'activer un matériau à mémoire de forme dans le dispositif d'annuloplastie ajustable et lui faire prendre une forme mémorisée.

2. Système d'activation d'annuloplastie selon la revendication 1, dans lequel le dispositif d'annuloplastie (2000, 2900, 2902) comprend :
un élément de corps ayant une extrémité proximale, une extrémité distale et une longueur s'étendant entre elles, l'élément de corps étant configuré pour être implanté dans le coeur d'un patient à la base ou près de la base d'une valve cardiaque ;
dans lequel l'élément de corps comprend :
une première portion, comprenant le matériau à mémoire de forme, et étant transformable d'une première configuration en une seconde configuration, en réponse à l'énergie d'activation ; et
une seconde portion, raccordée à la première portion, la seconde portion comprenant un matériau magnétique (2914, 2915) qui est sensible au champ magnétique ; et
dans lequel, quand il est en position à la base ou près de la base de la valve cardiaque et quand l'élément de corps se transforme de la première configuration à la seconde configuration, ledit élément de corps est adapté pour reprendre la forme d'un tissu cardiaque, de façon à exercer une force sur la base de la valve cardiaque.

3. Système d'activation d'annuloplastie selon la revendication 1, dans lequel l'élément distal (3214) est configuré ultérieurement pour fournir de l'énergie par champ électrique, de l'énergie thermique, de l'énergie par champ magnétique, de l'énergie par radiofréquence, de l'énergie par rayons X, de l'énergie par micro-ondes, de l'énergie par ultrasons, de l'énergie lumineuse, ou des combinaisons des précédentes.

4. Système activation d'annuloplastie selon la revendication 1, dans lequel l'extrémité distale (3208) comprend une portion flexible (3212).

5. Système d'activation d'annuloplastie selon la revendication 1, dans lequel l'élément distal (3214) comprend une pluralité de bandes magnétiques.

6. Système d'activation d'annuloplastie selon la revendication 1, dans lequel l'élément allongé (3200) comprend un orifice (3260) à l'extrémité proximale (3210), en communication fluidique avec l'extrémité distale (3208).

7. Système d'activation d'annuloplastie selon la revendication 1, dans lequel l'élément allongé (3200) est configuré pour fournir de l'énergie au dispositif d'annuloplastie (2000).

8. Système d'activation d'annuloplastie selon la revendication 2, dans lequel le matériau magnétique produit une force comprise entre approximativement 0,89 N (0,2 livre) de force et approximativement 8,9 N (2,0 livres) de force, à une distance comprise entre 1 millimètre et 10 millimètres.

9. Système d'activation d'annuloplastie selon la revendication 8, dans lequel le matériau magnétique produit un champ magnétique compris entre environ 100 Gauss et environ 10 000 Gauss.

10. Système d'activation d'annuloplastie selon la revendication 2, dans lequel le matériau magnétique produit une force comprise entre 0,45 N (0,1 livre) de force et 13,35 N (3,0 livres) de force, à une distance comprise entre 0,5 millimètre et vingt millimètres et a une gamme comprise entre environ 100 Gauss et environ 700 Gauss.

11. Système d'activation d'annuloplastie selon la revendication 2, dans lequel le matériau magnétique comprend des aimants de forme cylindrique.

12. Système d'activation d'annuloplastie selon la revendication 11, dans lequel les aimants ont un diamètre compris entre approximativement 0,2 mm et approximativement 0,4 mm et une hauteur comprise entre environ 0,2 mm et environ 0,4 mm.

13. Système d'activation d'annuloplastie selon la revendication 2, dans lequel le matériau magnétique comprend une forme de tige, une forme sphérique, une forme de disque, une forme cubique, ou une forme de bande.

14. Système d'activation d'annuloplastie selon la revendication 2, dans lequel le dispositif d'annuloplastie (2000) est en forme de bague.

15. Système d'activation d'annuloplastie selon la revendication 2, dans lequel le dispositif d'annuloplastie (2000) est en forme de C.

16. Système d'activation d'annuloplastie selon la revendication 2, dans lequel le dispositif d'annuloplastie (2000) est en forme de D.
